# EUROPEAN PATENT APPLICATION

(11) **EP 3 978 517 A1**
(43) Date of publication of application: **06.04.2022**
(21) Application number: 21180474.5
(22) Date of filing: 18.03.2010
(51) Int. Cl.: C07K 14/47, A61P 25/28, A61K 39/00, A61P 3/10, A61P 21/02, A61P 25/00, A61P 25/16, A61P 27/02, A61P 27/06, A61P 35/00, A61P 37/02, A61P 37/04, A61P 31/18

(54) **METHOD FOR THERAPEUTIC USE**

(30) Priority: 18.03.2009 US 161165 P; 29.10.2009 US 256028 P
(62) Divisional of application: 10710017.4
(71) Applicant: AC Immune SA, 1015 Lausanne (CH)
(72) Inventor: Pfeifer, Andrea, 1806 St. Légier (CH); Muhs, Andreas, 1009 Pully (CH); Pihlgren, Maria, 1052 Mont-sur-Lausanne (CH)
(74) Representative: Vossius & Partner Patentanwälte Rechtsanwälte mbB

(57) **Abstract**

The present invention relates to an antigenic composition comprising an adjuvant and a tau peptide antigen modified by four palmitic acid moieties inserted into the lipid bilayer of a liposome and presented in a highly repetitive array on the surface of the liposome, for use in the treatment of a tau-associated disease or disorder, in a patient having a T-cell deficiency, wherein
(i) the tau peptide is the peptide shown in SEQ ID NO:5; and
(ii) the adjuvant is MPLA.

SEQUENCE LISTING-Rule 13ter

<110>  AC Immune SA

<120>  METHOD FOR THERAPEUTIC USE

<130>  R1512 EP/1 BS

<150>  61/161,165
<151>  2009-03-18

<150>  61/256,028
<151>  2009-10-29

<160>  14

<170>  PatentIn version 3.4

<210>  1
<211>  30
<212>  PRT
<213>  Homo sapiens

<220>
<221>  VARIANT
<222>  (18)..(18)
<223> /replace="phosphorylated serine"

<220>
<221>  VARIANT
<222>  (26)..(26)
<223> /replace="phosphorylated serine"

<400>  1

Arg Glu Asn Ala Lys Ala Lys Thr Asp His Gly Ala Glu Ile Val Tyr
1               5                   10                  15

Lys Ser Pro Val Val Ser Gly Asp Thr Ser Pro Arg His Leu
            20                  25                  30

<210>  2
<211>  16
<212>  PRT
<213>  Homo sapiens

<220>
<221>  VARIANT
<222>  (14)..(14)
<223> /replace="phosphorylated tyrosine"

<400>  2

Arg Gln Glu Phe Glu Val Met Glu Asp His Ala Gly Thr Tyr Gly Leu

1                   5                   10                  15

<210>  3
<211>  16
<212>  PRT
<213>  Homo sapiens

<220>
<221>  VARIANT
<222>  (7)..(7)
<223> /replace="phosphorylated threonine"

<220>
<221>  VARIANT
<222>  (9)..(9)
<223> /replace="phosphorylated serine"

<400>  3

Pro Gly Ser Arg Ser Arg Thr Pro Ser Leu Pro Thr Pro Pro Thr Arg
1               5                   10                  15

<210>  4
<211>  16
<212>  PRT
<213>  Homo sapiens

<220>
<221>  VARIANT
<222>  (7)..(7)
<223> /replace="phosphorylated serine"

<220>
<221>  VARIANT
<222>  (10)..(10)
<223> /replace="phosphorylated threonine"

<400>  4

Gly Tyr Ser Ser Pro Gly Ser Pro Gly Thr Pro Gly Ser Arg Ser Arg
1               5                   10                  15

<210>  5
<211>  16
<212>  PRT
<213>  Homo sapiens

<220>
<221>  VARIANT
<222>  (4)..(4)
<223> /replace="phosphorylated serine"

<220>
<221>  VARIANT
<222>  (12)..(12)
<223> /replace="phosphorylated serine"

<400>  5

Val Tyr Lys Ser Pro Val Val Ser Gly Asp Thr Ser Pro Arg His Leu
1               5                   10                  15


<210>  6
<211>  18
<212>  PRT
<213>  Homo sapiens


<220>
<221>  VARIANT
<222>  (4)..(4)
<223> /replace="phosphorylated serine"


<220>
<221>  VARIANT
<222>  (9)..(9)
<223> /replace="phosphorylated serine"

<400>  6

Gly Asp Thr Ser Pro Arg His Leu Ser Asn Val Ser Ser Thr Gly Ser
1               5                   10                  15


Ile Asp




<210>  7
<211>  17
<212>  PRT
<213>  Homo sapiens


<220>
<221>  VARIANT
<222>  (3)..(3)
<223> /replace="phosphorylated serine"

<220>
<221>  VARIANT
<222>  (6)..(6)
<223> /replace="phosphorylated threonine"

<220>
<221>  VARIANT
<222>  (13)..(13)
<223> /replace="phosphorylated threonine"

<220>
<221>  VARIANT
<222>  (15)..(15)
<223> /replace="phosphorylated serine"

<400>  7

Pro Gly Ser Pro Gly Thr Pro Gly Ser Arg Ser Arg Thr Pro Ser Leu
1               5                   10                  15

Pro

<210>  8
<211>  15
<212>  PRT
<213>  Homo sapiens
<220><223>  antigenic peptide Aß 1-15

<400>  8

Asp Ala Glu Phe Arg His Asp Ser Gly Tyr Glu Val His His Gln
1               5                   10                  15

<210>  9
<211>  16
<212>  PRT
<213>  Homo sapiens
<220><223>  antigenic peptide Aß 1-16

<400>  9

Asp Ala Glu Phe Arg His Asp Ser Gly Tyr Glu Val His His Gln Lys
1               5                   10                  15

<210>  10
<211>  15
<212>  PRT
<213>  Homo sapiens
<220><223>  antigenic peptide Aß 1-16(Δ14)

<400>  10

Asp Ala Glu Phe Arg His Asp Ser Gly Tyr Glu Val His Gln Lys
1               5                   10                  15

<210>  11
<211>  8
<212>  PRT
<213>  Homo sapiens
<220><223>  antigenic peptide Aß 4-11

<400>  11

Phe Arg His Asp Ser Gly Tyr Glu
1               5

<210>  12
<211>  13
<212>  PRT
<213>  Homo sapiens
<220><223>  antigenic peptide Aß 22-35

<400>  12

Glu Asp Val Gly Ser Asn Lys Gly Ala Ile Ile Gly Leu
1               5                   10

<210>  13
<211>  12
<212>  PRT
<213>  Homo sapiens
<220><223>  antigenic peptide Aß 29-40

<400>  13

Gly Ala Ile Ile Gly Leu Met Val Gly Gly Val Val
1               5                   10

<210>  14
<211>  5
<212>  PRT
<213>  Homo sapiens
<220><223>  antigenic peptide Aß 1-5

<400>  14

Asp Ala Glu Phe Arg
1               5

## Description

### FIELD OF THE INVENTION

The present invention is related to methods for inducing a T-cell independent immune response in an individual, particularly animal or human upon treatment of a disease or disorder in a patient, particularly diseases and disorders which are caused by or associated with amyloid or amyloid-like proteins or with tau or tau-like proteins including Alzheimer's Disease.

In particular, the present invention is related to methods for the therapeutic and diagnostic use in the treatment of diseases and disorders which are caused by or associated with amyloid or amyloid-like proteins including amyloidosis, a group of disorders and abnormalities associated with amyloid protein such as Alzheimer's Disease. The present invention is further related to methods and compositions for the therapeutic and diagnostic use in the treatment of diseases and disorders which are caused by or associated with neurofibrillary tangles. In particular, the invention relates to methods and compositions for the therapeutic and diagnostic use in the treatment of tauopathies including Alzheimer's Disease (AD).

### BACKGROUND OF THE INVENTION

Antigens are classically divided into T-dependent and T-independent antigens (Alugupulli 2008). Proteins and peptides are referred to as T-dependent antigens as they induce antigen-specific T cells that help B cells by providing cognate and noncognate interactions. Many vaccines are T-dependent antigens. T-dependent antigens generally require multiple boosters to generate an antibody response

Antigens that induce antibody responses without T cell help are referred to as T-independent antigens (Bachmann and Zinkernagel 1997). These antigens induce antibody response in the absence of T helper cells in vivo, for example in athymic nude (nu/nu) mice. T-independent antigens are typically antigens that cannot stimulate a T-cell response as they cannot be processed and presented by MHC-II molecules. LPS and bacterial polysaccharides are typical T-cell-independent antigens. In addition, some protein of certain viruses and bacteria can stimulate an antibody response in the absence of T cells, most likely due to their repetitive structure that can efficiently crosslink the BCR. B-cell expansion and strong antibody titers are induced much faster with T-independent than T-dependent antigens.

Generally, B-cells are not fully activated to proliferate and produce immunoglobulin (Ig) by simply cross-linking the B-cell surface Ig. To achieve optimum antibody production, a second signal is required, namely that provided by a T-helper cell. Antigens bind to the Ig on the B-cell surface, which is internalized and then presented in the context of MHC Class II molecules to antigen specific T-helper cells. Also the B-cell expresses CD40 as well as B7 (CD86), interacting with CD40 ligand (CD40L) and CD28, respectively, on the T-cell, thereby inducing cytokine secretion by the T-cell that further modulate the B-cell response. These cytokines trigger B-cell proliferation and differentiation into antibody-producing cells (plasma cells). This isotype switching to IgG, IgA or IgE is known as Class Switch Recombination (CSR). Once this switch has occurred, a particular B-cell can no longer make the other isotypes, IgM or IgD.

While the vast majority of antigens require T-cell help for B-cell activation and is therefore called T-cell help dependent antigens (TD antigens), a minority of antigens can induce antibodies independent of T-help, called TI-antigens (Mond JJ, Vos Q, Lees A, Snapper CM. T-cell independent antigens. Curr Opin Immunol 1995; 7:349-54.) Based on their immunogenicity in congenitally athymic (nu/nu) mice and mice with an X linked immune B-cell defect (xid mice, lacking the function of Burton's tyrosine kinase Btk), TI-antigens are classically further subdivided into TI-type 1 antigens (TI-1) and TI-type 2 (TI-2) antigens. TI-1 antigens stimulate excellent antibody responses in nu/nu mice and in xid-mice. TI-2 antigens also stimulate excellent antibody responses in nu/nu mice, but not in xid-mice. TI-1 antigens contain polyclonal B-cell activators, which are typically lipopolysaccharides (LPS). In contrast, TI-2 antigens are typically antigens consisting of repetitive biochemical structures, such as polymeric protein antigens or capsular polysaccharides of bacteria.

Most peptide or protein vaccines are based on the principle that both B- and T-cells are activated and the antibody response is T-cell-dependent. This can cause problems in patients which suffer from T-cell deficiencies due to age, immunosuppression or diseases.

In aging immunes system pronounced changes occur in terms of T-cell number and quality, which are a direct consequence of thymic involution. The thymus, a central lymphoid organ that lies in the upper anterior thorax just above the heart, is responsible for the development, selection and output of mature naive T-cells into the periphery in a process called thymopoiesis. With progressive ageing, the thymus involutes. As a result, the output of naive T-cells declines dramatically with age. The reduced thymic output together with a life-long exposure to antigens leads to a shrinkage of the naive T-cell pool in elderly persons and consequently to limitations in the host's ability to respond to new antigens. This is particularly disturbing when old individuals contract newly emerging diseases, such as the severe acute respiratory syndrome (SARS), which killed 50% of infected persons over 50 years of age. Additionally, the naive T-cells found in old age have resided for decades in the periphery and have been exposed to a series of factors, such as oxidative stress and limited concentrations of survival factors, that may have a negative impact on cell function. Naive CD4+ T cells from aged animals, compared with cells from young mice, produce significantly less IL-2, expand poorly and give rise to fewer effectors, with a less activated phenotype and reduced ability to produce cytokines. However, newly generated CD4+ T-cells in old animals respond strongly to antigens in vivo and in vitro, are able to expand, produce IL-2 and exhibit cognate helper function. Thus, age-related defects in response to antigenic stimulation may, at least in part, be due to the chronological age of CD4+ T-cells.

Memory CD4+ T-cells are pivotal in controlling humoral and cellular responses, and so their longevity and response to vaccination are critical for the maintenance of protective immunity. Memory T-cells generated from aged naive T-cells survive and persist well in vivo but they are markedly defective in their proliferation and cytokine secretion during recall responses, whereas memory cells generated in young individuals retain function for extended periods of time as their host ages These data, from animal studies, have recently been confirmed in humans. Healthy elderly individuals are able to mount a CD4+ T-cell response comparable to that observed in younger individuals when vaccinated against influenza, but they exhibit an impaired longterm CD4+ T-cell immune response to the influenza vaccine.

T-cell deficiencies do, however, not only occur as a consequence of ageing, but can also be found in immunosuppressed patients or in patients which suffer from certain diseases such as, for example, HIV, cancer or other malignancies.

### SUMMARY OF THE INVENTION

In a particular embodiment of the present invention an antigenic composition has been generated comprising a palmitoylated peptide presented on liposomes combined with MPLA, which vaccine is T-cell independent. The peptide is in beta-sheet conformation. This vaccine induces rapid and strong antibody responses. It is not dependent on another carrier protein and can induce antibody responses in the absence of T-cells, as demonstrated by antibody induction in nude mice. Finally, it induces a strong and effective antibody response in elderly mice despite the T-cell deficiency at this old age (Miller R.A, et al., 1996; Adolfsson O, et all, 2001; Linton P.J., et al. 1996).

### Summary of characteristics of the vaccine

- Specific conformation (beta-sheet)
- Strong response (1000000 ng/ml at 7 days after immunization)
- Fast rapid response (7 days after first immunization)
- No need for T-cell help
- No need for carrier protein
- Effectiveness in elderly mice, despite T-cell deficiencies at this old age

Aβ antigenic compositions known in the prior art, such as AN1792 from the company Elan Inc (CA; USA), are usually typical T-dependent antigens (Aβ1-42) administered with a strong Th1 driving adjuvant (QS21). An initial study of AN1792 in Alzheimer's Disease patients showed promising efficacy, with a slower rate of cognitive decline in patients who had received vaccination than in placebo-treated patients (Gilman et al. 2005). However 6% of treated patients developed meningoencephalitis a reaction considered to be due to a T-cell-mediated response against full length Aβ1-42 (Orgogozo et al. 2003). Although reversible in most patients this inflammatory response led to discontinuation of the clinical trial.

What is therefore needed, are methods wherein the use of an antigen, such as, for example, an anti-Aβ antigen or an anti-tau antigen, induces an antibody response through direct activaton of B-cells in the absence of antigen-specific, i.e. Aβ- or tau-specific, T-cells for the treatment, alleviation of the symptoms, delaying onset or prevention of diseases and disorders, such as diseases and disorders which are caused by or associated with amyloid or amyloid-like proteins including amyloidosis, a group of disorders and abnormalities associated with amyloid protein such as Alzheimer's Disease, or diseases and disorders which are caused by or associated with protein tau including tauopathies, in patients with a T-cell deficiency, particularly patients which are depleted of CD4 T-cells. Particularly, palmitoylated Aβ peptide or tau peptide presented by liposomes in combination with MPLA induce Aβ-specific antibodies without the need for T-cells help and without the need for a carrier protein providing T cell help.

The present invention provides a method for the therapeutic and diagnostic use in the treatment, alleviation of the symptoms or prevention of diseases and disorders comprising administering to a patient a T-cell independent antigenic composition comprising a modified antigen, wherein said antigen is presented in a highly repetitive array on the surface of a liposome. In particular, said diseases and disorders are caused by or associated with amyloid or amyloid-like proteins including amyloidosis, a group of disorders and abnormalities associated with amyloid protein such as Alzheimer's Disease, or diseases and disorders which are caused by or associated with protein tau including tauopathies, in a patient, particularly an immune tolerant patient, particular in a patient suffering from a T-cell deficiency, particularly a T-cell deficiency which is caused by a depletion within said patients of CD4 T-cells and/or a reduced expression of CD14 and/or the CD40L on CD4 T-cells such as, for example, an immunocompromised patient or a patient having an autoimmune disease.

Moreover, it was now surprisingly found within the scope of the present invention that diseases in patients, which are in need of a T-cell independent immune response, particularly in immune tolerant patients, particularly in patients suffering from a T-cell immunodeficiency disease, can be avoided in an individual, particularly animal or human, wherein the individual can be either vaccinated before the onset of the disease, such as, for example, an amyloid- or tau protein-associated disease or, in case of suffering from an amyloid- or tau protein-associated disease or condition, the individual can be treated by the method described herein.

The technical problem underlying the present invention is the provision of a method for the therapeutic and diagnostic use of an antigenic composition in the treatment, alleviation of the symptoms or prevention of diseases and disorders in a patient, particularly a patient in need of a T-cell independent immune response such as, for example, an immune tolerant patient or a T-cell activated patient, wherein a T-cell independent immune response is induced in the treated individual. In particular, diseases and disorders to be treated within the scope of the present invention are, for example, diseases and disorders which are caused by or associated with amyloid or amyloid-like proteins including amyloidosis, a group of disorders and abnormalities associated with amyloid protein such as Alzheimer's Disease, or diseases and disorders which are caused by or associated with protein tau-including tauopathies.

The technical problem is solved by the provision of the embodiments characterized in the claims.

The present invention thus relates in a first embodiment to an antigenic composition comprising a modified antigen for inducing a T-cell independent immune response upon treatment of a disease, condition or disorder in a patient, particularly an animal or human patient, particularly a patient in need of such a T-cell independent response such as, for example, , an immune tolerant patient or a T-cell activated patient wherein said antigen is presented on the surface of a liposome.

In one embodiment, the antigenic composition of the invention as described herein is effective as an immune stimulant.

In a specific embodiment of the invention, said antigen is a peptide antigen, which is presented in a highly repetitive array on the surface of the liposome. In a further specific embodiment, said antigen does not contain a T-cell epitope.

In one embodiment of the invention, the antigenic composition of the invention as described herein is used for treating an immune tolerant patient or a T-cell activated patient, particularly a immunocompromised patient, particularly a patient suffering from an autoimmune disease, particularly a patient who suffers from a T-cell deficiency, particularly a T-cell deficiency, which is caused by a depletion within said patients of CD4 T-cells and/or a reduced expression of CD14 and/or the CD40L on CD4 T-cells.

In one embodiment, the invention relates to the antigenic composition of the invention and as described herein, wherein said T-cell deficiency is caused by age, immunosuppression or disease, particularly a disease selected from the group of HIV, cancer or other malignancies including, for example, inflammatory diseases which are treated with immunosuppressive and chemotherapeutic compounds, respectively, leading to an immune-suppressed status of the patients such as, for example, Rheumatoid Arthritis; Psoriasis, Systemic Lupus Erythrematosis,Wegener's Granulamatosis, etc.

In one embodiment, the antigenic composition of the invention and as described herein comprises a conformational antigen, wherein all or part of said antigen is in a beta-sheet conformation. In a specific embodiment, said antigenic peptide is modified such that it is capable of maintaining and stabilizing a defined conformation of the antigen, particularly a conformation which is characterized by a balanced proportion of random coil, α-helical and β-sheet portions. This defined conformation leads to the induction of a strong and highly specific immune response, particularly a T-cell independent immune response, upon introduction into an animal or a human.

In particular, the antigenic composition of the invention and as described herein may comprise a conformational antigen, wherein more than 30%, particularly mor than 40%, particularly more than 50%, particularly more than 60%, particularly more than 70%, particularly more than 80%, particularly more than 90%, particularly more than 95% and up to 100% is in a beta-sheet conformation.

In one embodiment, the antigenic composition of the invention and as described herein comprises a peptide antigen, which is modified by a lipophilic or hydrophobic moiety, particularly a fatty acid, a triglyceride, a diglyceride, a steroid, a sphingolipid, a glycolipid or a phospholipid that facilitates insertion into the lipid bilayer of the liposome.

In a specific embodiment, the the lipophilic or hydrophobic moiety is a fatty acid, particularly a fatty acid with a carbon back bone of at least 10 carbon atoms, particularly a palmitic acid.

In another specific embodiment of the invention, the antigenic composition of the invention and as described herein comprises a peptide antigen comprising two palmitic acid moieties, particularly four palmitic acid moieties.

In still another specific embodiment of the invention, the dimension of the lipophilic or hydrophobic moiety in combination with the overall net charge of the antigenic peptide and of the carrier/adjuvant to which the peptide becomes attached to, incorporated or reconstituted in, is such that the antigenic peptide is exposed, stabilized and presented in a highly biologically active conformation, which allows the immune system of the target organism to freely interact with the antigenic determinants contained in the antigenic construct in its exposed, stabilized and highly biologically active conformation, which leads to a strong immune response.

In one embodiment, the antigenic composition of the invention and as described herein, comprises a liposome preparation and an adjuvant, particularly lipid A, particularly detoxified lipid A, such as monophosphoryl or diphosphoryl lipid A or alum.

In one embodiment, the antigenic composition of the invention and as described herein, comprises an antigenic construct, particularly an Aβ antigenic construct, comprising a single or repetitive Aβ peptide fragment derived from the N-terminus of the Aβ peptide.

In a specific embodiment of the invention, the N-terminus of the Aβ peptide comprises
a) Aβ 1-30;
b) Aβ 1-20; or
c) Aβ 1-16;

In another specific embodiment of the invention, the Aβ peptide fragment comprises between
a) 4 and 20 amino acids;
b) 5 and 16 amino acids;
c) 6 and 12 amino acids; or
d) 4 and 8 amino acids.

In still another specific embodiment of the invention, the Aβ peptide fragment is
a) Aβ₁₋₁₅;
b) Aβ₁₋₁₆;
c) Aβ₁₋₅.

In one embodiment, the antigenic composition of the invention and as described herein comprises an antigenic construct comprising an antigenic peptide, which is a phospho-peptide mimicking a major pathological phospho-epitope of protein tau, particularly a human protein tau.

In a specific embodiment of the invention, said tau protein has an amino acid sequence identity to
a) SEQ ID NO: 2 of at least 95%, and has substantially the same immunogenic activity as said antigenic peptide of SEQ ID NO: 2, wherein the amino acid residue corresponding to amino acid residue 18 (P-Tyr₁₈) of SEQ ID NO: 2 is phosphorylated (T1);
b) SEQ ID NO: 3 of at least 95%, and has substantially the same immunogenic activity as said antigenic peptide of SEQ ID NO: 3, wherein at least one, particularly at least 2 of amino acid residues corresponding to amino acid residues 212 (P-Thr₂₁₂) and 214 (P-Ser₂₁₄) of SEQ ID NO: 3 are phosphorylated;
c) SEQ ID NO: 4 of at least 95%, and has substantially the same immunogenic activity as said antigenic peptide of SEQ ID NO: 4, wherein at least one, particularly at least 2 of amino acid residues corresponding to amino acid residues 202 (P-Ser₂₀₂) and 205 (P-Thr₂₀₅) of SEQ ID NO: 4 are phosphorylated;
d) SEQ ID NO: 5 of at least 95%, and has substantially the same immunogenic activity as said antigenic peptide of SEQ ID NO: 5, wherein at least one, but especially all of amino acid residues corresponding to amino acid residues 396 (P-Ser₃₉₆) and 404 (P-Ser₄₀₄) of SEQ ID NO: 5 are phosphorylated;
e) SEQ ID NO: 6 of at least 95%, and has substantially the same immunogenic activity as said antigenic peptide of SEQ ID NO: 6, wherein at least one, but especially all of amino acid residues corresponding to amino acid residues 404 (P-Ser₄₀₄) and 409 (P-Ser₄₀₉) of SEQ ID NO: 6 are phosphorylated;
f) SEQ ID NO: 7 of at least 95%, and has substantially the same immunogenic activity as said antigenic peptide of SEQ ID NO: 7, wherein at least one, particularly at least 2, particularly at least 3, but especially all of amino acid residues corresponding to amino acid residues 202 (P-Ser₂₀₂), 205 (P-Thr₂₀₅), 212 (P-Thr₂₁₂), and 214 (P-Ser₂₁₄) of SEQ ID NO: 7 are phosphorylated.

In another specific embodiment, the antigenic composition of the invention and as described herein comprises a tau protein, which has an amino acid sequence of
a) SEQ ID NO: 2;
b) SEQ ID NO: 3;
c) SEQ ID NO: 4;
d) SEQ ID NO: 5;
e) SEQ ID NO: 6; or
f) SEQ ID NO: 7.

In still another embodiment, the invention relates to the antigenic composition of any of the previously described embodiments comprising a modified antigen for use in the treatment of a disease, condition or disorder in a patient, particularly an animal or human patient, particularly a patient in need of a T-cell independent response such as, for example, an immune tolerant patient or a T-cell activated patient, particularly a immunocompromised patient, particularly a patient suffering from an autoimmune disease, particularly a patient who suffers from a T-cell deficiency, particularly a T-cell deficiency, which is caused by a depletion within said patients of CD4 T-cells and/or a reduced expression of CD14 and/or the CD40L on CD4 T-cells, to overcome or reduce immune tolerance in said patient, wherein said antigen is presented in a highly repetitive array on the surface of a liposome and wherein said patient is suffering from a disease or disorder which is caused by or associated with amyloid or amyloid-like proteins.

In still another embodiment, the invention relates to the antigenic composition of any of the previously described embodiments comprising a modified antigen for use in the treatment of a disease, condition or disorder in a patient, particularly an animal or human patient, particularly a patient in need of a T-cell independent response such as, for example, an immune tolerant patient or a T-cell activated patient, particularly a immunocompromised patient, particularly a patient suffering from an autoimmune disease, particularly a patient who suffers from a T-cell deficiency, particularly a T-cell deficiency, which is caused by a depletion within said patients of CD4 T-cells and/or a reduced expression of CD14 and/or the CD40L on CD4 T-cells, to overcome or reduce immune tolerance in said patient, wherein said antigen is presented in a highly repetitive array on the surface of a liposome and wherein said patient is suffering from a disease or disorder which is caused by or associated with tau or tau-like proteins.

In a specific embodiment of the invention, the amyloid-associated disease or disorder is characterized by a loss of cognitive memory capacity in an individual, particularly animal or human.

In another specific embodiment of the invention, said disease is a disease or disorder is a neurological disease or disorder, particularly a disease or disorder from the amyloidosis group, particularly a disease or disorder selected from the group consisting of Alzheimer's Disease (AD), mild cognitive impairment (MCI), Lewy body dementia, Down's syndrome, hereditary cerebral hemorrhage with amyloidosis (Dutch type); the Guam Parkinson-Dementia complex; progressive supranuclear palsy, multiple sclerosis; Creutzfeld-Jacob disease, Parkinson's disease, HIV-related dementia, ALS (amyotropic lateral sclerosis), Adult Onset Diabetes; senile cardiac amyloidosis; endocrine tumors, stroke, traumatic brain injury, macular degeneration and glaucoma.

In a specific embodiment, said disease is Alzheimer's Disease.

In still another specific embodiment of the invention, the tau-associated disease or disorder is a neurodegenerative disorder, particularly a neurodegenerative disease or disorder, which is caused by or associated with the formation of neurofibrillary lesions.

In a specific embodiment of the invention, neurodegenerative disease or disorder is a tauopathy, particularly selected from the group consisting of Alzheimer's Disease, Creutzfeld-Jakob disease, Dementia pugilistica, Down's Syndrome, Gerstmann-Straussler-Scheinker disease, inclusion-body myositis, and prion protein cerebral amyloid angiopathy, traumatic brain injury, amyotrophic lateral sclerosis/parkinsonism-dementia complex of Guam, Non-Guamanian motor neuron disease with neurofibrillary tangles, argyrophilic grain dementia, corticobasal degeneration, diffuse neurofibrillary tangles with calcification, frontotemporal dementia with parkinsonism linked to chromosome 17, Hallevorden-Spatz disease, multiple system atrophy, Niemann-Pick disease, Type C, Pick's disease, progressive subcortical gliosis, progressive supranuclear palsy, Subacute sclerosing panencephalitis, Tangle only dementia, Postencephalitic Parkinsonism, Myotonic dystrophy.

In a specific embodiment of the invention, said neurodegenerative disorder is Alzheimer's Disease.

In one aspect of the invention, the antigenic composition of any of the previously described embodiments does not show any adverse inflammatory effects.

In one embodiment, the invention relates to a method of using an antigenic composition comprising a modified antigen according to any of the previously described embodiments for the preparation of a medicament for inducing a T-cell independent immune response upon treatment of a disease, condition or disorder in a patient, particularly an animal or human patient, particularly a patient in need of such a T-cell independent response, wherein said antigen is presented on the surface of a liposome, particularly in a highly repetitive array. In one embodiment of the invention said antigenic composition is effective as an immune stimulant.

In one embodiment, the invention relates to a method of using an antigenic composition comprising a modified antigen according to any of the previously described embodiments for the preparation of a medicament for use in the treatment of an immune tolerant patient or a T-cell activated patient, particularly an animal or a human, particularly a immunocompromised patient, particularly a patient suffering from an autoimmune disease, particularly a patient who suffers from a T-cell deficiency, particularly a T-cell deficiency, which is caused by a depletion within said patients of CD4 T-cells and/or a reduced expression of CD14 and/or the CD40L on CD4 T-cells, to overcome or reduce immune tolerance in said patient or to avoid an overstimulation of the T-cell response in said patient, wherein said antigen is presented in a highly repetitive array on the surface of a liposome.

Also comprised by the present invention is a method for inducing a T-cell independent immune response upon treatment of a disease, condition or disorder in a patient, particularly an animal or human patient, particularly a patient in need of such a T-cell independent response, wherein said antigen is presented on the surface of a liposome, particularly in a highly repetitive array. In one embodiment of the invention said antigenic composition is effective as an immune stimulant.

Also comprised by the present invention is a method for overcoming or reducing an immune tolerance in an immune tolerant patient or to avoid an overstimulation of the T-cell response in a T-cell activated patient, upon treatment of a disease or disorder in said patient, particularly an animal or a human patient, particularly a immunocompromised patient, particularly a patient suffering from an autoimmune disease, particularly a patient who suffers from a T-cell deficiency, particularly a T-cell deficiency, which is caused by a depletion within said patients of CD4 T-cells and/or a reduced expression of CD14 and/or the CD40L on CD4 T-cells, comprising administering to said patient an antigenic composition comprising a modified antigen according to any of the previously described embodiments, wherein said antigen is presented in a highly repetitive array on the surface of a liposome; and optionally the further step of determining the immune response in said individual by measuring the antibody concentration of IgG and/or IgM. The present invention further relates in another embodiment to a method for the treatment, alleviation of the symptoms or prevention of a disease or condition, particularly an amyloid- or tau-protein associated disease or condition, in an immune tolerant patient or a T-cell activated patient, particularly an animal or human, which patient suffers from a T-cell deficiency, particularly a T-cell deficiency caused by a depletion of T-cells, particularly of CD4 T-cells such as, for example, an immunocompromised patient or a patient having an autoimmune disease, comprising administering to said patient an antigenic composition comprising a modified antigen according to any of the previously described embodiments, wherein said antigen is presented in a highly repetitive array on the surface of a liposome and wherein a T-cell independent immune response is induced in the treated patient.

The present invention thus further relates to a method for inducing a T-cell independent immune response in an immune tolerant patient or a T-cell activated patient, particularly to a method for the treatment, alleviation of the symptoms, delaying onset or prevention of a disease or disorder in such an immune tolerant patient or a T-cell activated patient, which disease or disorder is caused by or associated with amyloid or amyloid-like proteins including amyloidosis, a group of disorders and abnormalities associated with amyloid protein such as Alzheimer's Disease, or diseases and disorders which are caused by or associated with protein tau including tauopathies, in an individual, particularly animal or human, particularly an animal or human who is depleted of T-cells, particularly of CD4 T-cells such as, for example, an immunocompromised patient or a patient having an autoimmune disease, comprising the following steps (a) administration of an antigenic construct, particularly an antigenic construct comprising a T-independent antigen, to said individual; and (b) determination of the immune response in said individual, particularly by measuring the antibody concentration of IgG.

In a particular embodiment of the invention, said individual is an elderly human patient depleted of T-cells, particularly of CD4 T-cells, particularly an immunocompromised patient or a patient having an autoimmune disease.

In another embodiment of the invention, said individual is an immunosuppressed human patient, who is depleted of T-cells, particularly of CD4 T-cells.

In still another embodiment of the invention, said individual is a human patient, who is depleted of T-cells, particularly of CD4 T-cells and suffers from an immunodeficiency disease, particularly from HIV.

In still another embodiment of the invention, said individual is a human patient, who has cancer or another malignancy and is depleted of T-cells, particularly of CD4 T-cells.

In still another embodiment of the invention, said individual is a human patient, who is depleted of T-cells, particularly of CD4 T-cells and suffers from an autoimmune disease.

The present invention further relates in another embodiment to a method for inducing a T-cell independent immune response, particularly an Aβ immune response or a tau-related immune respone, in an individual, particularly an animal or human, particularly an individual in need of such a T-cell independent Aβ immune response, particularly an immune tolerant individual or patient, who is depleted of T-cells, particularly of CD4 T-cells such as, for example, an immunocompromised patient or a patient having an autoimmune disease, comprising the following steps (a) administration of an antigentic construct, particularly an Aβ- or tau-related antigenic construct, particularly an Aβ- or tau-related antigenic construct comprising a T-independent antigen, to said individual; and (b) determination of the immune response in said individual, particularly by measuring the antibody concentration of IgG.

In another embodiment, the invention relates to a method according to the previously described embodiment, wherein said Aβ- or tau-related antigen induces an antibody response in the absence of T-helper cells *in vivo.*

In another embodiment of the present invention a method according to any of the previously described embodiments is provided, wherein said Aβ- or tau-related immune response is an IgG response.

In one embodiment as described herein a method according to any of the previously described embodiments is provided, wherein the immune tolerant individual or patient or the T-cell activated patient, particularly animal or human, particularly an animal or human who is depleted of T-cells, particularly of CD4 T-cells, does not show any adverse inflammatory effect.

In another embodiment of the present invention, a method according to any of the previously described embodiments is provided, wherein the immune tolerant individual or patient, particularly animal or human, is T-cell deficient, particularly CD4 T-cell deficient such as, for example, an immunocompromised patient or a patient having an autoimmune disease.

In a further embodiment, a method according to any of the previously described embodiments is provided, wherein the Aβ antigenic construct comprises a single or repetitive Aβ peptide fragment derived from the N-terminus of the Aβ peptide.

In another embodiment of the present invention, a method according to any of the previously described embodiments is provided, wherein the N-terminus of the Aβ peptide comprises Aβ 1-30; Aβ 1-20; or Aβ 1-16.

In a further embodiment, a method according to any of the previously described embodiments is provided, wherein the Aβ peptide fragment comprises between 4 and 20 amino acids; between 5 and 16 amino acids; between 6 and 12 amino acids; or between 4 and 8 amino acids.

In particular, the present invention relates in another embodiment to a method according to the previous embodiment, wherein said Aβ peptide fragment is Aβ_{1-15;} Aβ₁₋₁₆; or Aβ₁₋₅.

In one embodiment, the invention relates to a method according to any of the previously described embodiment, wherein the antigenic composition comprises an antigenic construct comprising an antigenic peptide, which is a phospho-peptide mimicking a major pathological phospho-epitope of protein tau, particularly a human protein tau.

In a specific embodiment of the invention, said tau protein has an amino acid sequence identity to
a) SEQ ID NO: 2 of at least 95%, and has substantially the same immunogenic activity as said antigenic peptide of SEQ ID NO: 2, wherein the amino acid residue corresponding to amino acid residue 18 (P-Tyr₁₈) of SEQ ID NO: 2 is phosphorylated (T1);
b) SEQ ID NO: 3 of at least 95%, and has substantially the same immunogenic activity as said antigenic peptide of SEQ ID NO: 3, wherein at least one, particularly at least 2 of amino acid residues corresponding to amino acid residues 212 (P-Thr₂₁₂) and 214 (P-Ser₂₁₄) of SEQ ID NO: 3 are phosphorylated;
c) SEQ ID NO: 4 of at least 95%, and has substantially the same immunogenic activity as said antigenic peptide of SEQ ID NO: 4, wherein at least one, particularly at least 2 of amino acid residues corresponding to amino acid residues 202 (P-Ser₂₀₂) and 205 (P-Thr₂₀₅) of SEQ ID NO: 4 are phosphorylated;
d) SEQ ID NO: 5 of at least 95%, and has substantially the same immunogenic activity as said antigenic peptide of SEQ ID NO: 5, wherein at least one, but especially all of amino acid residues corresponding to amino acid residues 396 (P-Ser₃₉₆) and 404 (P-Ser₄₀₄) of SEQ ID NO: 5 are phosphorylated;
e) SEQ ID NO: 6 of at least 95%, and has substantially the same immunogenic activity as said antigenic peptide of SEQ ID NO: 6, wherein at least one, but especially all of amino acid residues corresponding to amino acid residues 404 (P-Ser₄₀₄) and 409 (P-Ser₄₀₉) of SEQ ID NO: 6 are phosphorylated;
f) SEQ ID NO: 7 of at least 95%, and has substantially the same immunogenic activity as said antigenic peptide of SEQ ID NO: 7, wherein at least one, particularly at least 2, particularly at least 3, but especially all of amino acid residues corresponding to amino acid residues 202 (P-Ser₂₀₂), 205 (P-Thr₂₀₅), 212 (P-Thr₂₁₂), and 214 (P-Ser₂₁₄) of SEQ ID NO: 7 are phosphorylated.

In another specific embodiment, the antigenic composition of the invention and as described herein comprises a tau protein, which has an amino acid sequence of
a) SEQ ID NO: 2;
b) SEQ ID NO: 3;
c) SEQ ID NO: 4;
d) SEQ ID NO: 5;
e) SEQ ID NO: 6; or
f) SEQ ID NO: 7.

The present invention relates in another embodiment to a method according to any of the preceding embodiments, wherein the antigen, particularly the Aβ- or tau-peptide antigen is presented reconstituted in a carrier such as, for example, a vesicle, a particulate body or molecule.

The present invention relates in another embodiment to a method according to any of the preceding embodiments, wherein the antigen, particularly the Aβ- or tau-peptide antigen is presented reconstituted in a liposome.

The present invention relates in another embodiment to a method according to any of the preceding embodiments, wherein the antigen, particularly the Aβ- or tau-peptide antigen is modified by a lipophilic or hydrophobic moiety that facilitates insertion into the lipid bilayer of the liposome carrier/adjuvant. The present invention relates in another embodiment to a method according to the previous embodiment, wherein the dimension of the lipophilic or hydrophobic moiety in combination with the overall net charge of the antigenic peptide and of the carrier/adjuvant to which the peptide becomes attached to, incorporated or reconstituted in such that the antigenic peptide is exposed, stabilized and presented in a highly biologically active conformation, which allows the immune system of the target organism to freely interact with the antigenic determinants contained in the antigenic construct in its exposed, stabilized and highly biologically active conformation, which leads to a strong immune response.

The present invention relates in another embodiment to a method according to any of the previous embodiments, wherein the lipophilic or hydrophobic moiety is a fatty acid, a triglycerides or a phospholipid, particularly a fatty acid with a carbon back bone of at least 10 carbon atoms, particularly a palmitic acid.

The present invention relates in another embodiment to a method according to the previous embodiment, wherein the antigen, particularly the Aβ- or tau-peptide antigen comprises two palmitic acid moieties, particularly four palmitic acid moieties.

The present invention relates in another embodiment to a method according to any of the previous embodiments, wherein the liposome preparation contains an adjuvant, particularly lipid A, particularly detoxified lipid A, such as monophosphoryl or diphosphoryl lipid A or alum.

The present invention relates in another embodiment to a method according to any one of the preceding embodiments, wherein the amyloid associated disease or condition is one selected from the group consisting of diseases including, but not limited to, neurological disorders such as Alzheimer's Disease (AD), including diseases or conditions characterized by a loss of cognitive memory capacity such as, for example, mild cognitive impairment (MCI), Lewy body dementia, Down's syndrome, hereditary cerebral hemorrhage with amyloidosis (Dutch type); the Guam Parkinson-Dementia complex; as well as other diseases which are based on or associated with amyloid-like proteins such as progressive supranuclear palsy, multiple sclerosis; Creutzfeld-Jacob disease, Parkinson's disease, HIV-related dementia, ALS (amyotropic lateral sclerosis), Adult Onset Diabetes; senile cardiac amyloidosis; endocrine tumors, stroke, traumatic brain injury, all ocular diseases and others, including macular degeneration and glaucoma, but particularly Alzheimer's Disease.

The present invention relates in another embodiment to a method according to the previous embodiment, wherein the amyloid-associated condition is characterized by a loss of cognitive memory capacity in an individual, particularly an immune tolerant individual or patient or a T-cell activated patient, particularly animal or human.

The present invention relates in another embodiment to a method according to the previous embodiment, wherein treatment of an immune tolerant individual or patient or a T-cell activated patient, particularly animal or human, suffering from an amyloid-associated condition characterized by a loss of cognitive memory capacity leads to an increase in the retention of cognitive memory capacity, particularly to a complete restoration of cognitive memory capacity.

In still another specific embodiment of the invention relates to a method according to any one of the preceding embodiments, wherein the tau-associated disease or disorder is a neurodegenerative disorder, particularly a neurodegenerative disease or disorder, which is caused by or associated with the formation of neurofibrillary lesions, particularly a tauopathy, particularly a tauopathy selected from the group consisting of Alzheimer's Disease, Creutzfeld-Jakob disease, Dementia pugilistica, Down's Syndrome, Gerstmann-Straussler-Scheinker disease, inclusion-body myositis, and prion protein cerebral amyloid angiopathy, traumatic brain injury, amyotrophic lateral sclerosis/parkinsonism-dementia complex of Guam, Non-Guamanian motor neuron disease with neurofibrillary tangles, argyrophilic grain dementia, corticobasal degeneration, diffuse neurofibrillary tangles with calcification, frontotemporal dementia with parkinsonism linked to chromosome 17, Hallevorden-Spatz disease, multiple system atrophy, Niemann-Pick disease, Type C, Pick's disease, progressive subcortical gliosis, progressive supranuclear palsy, Subacute sclerosing panencephalitis, Tangle only dementia, Postencephalitic Parkinsonism, Myotonic dystrophy, but particularly Alzheimer's Disease.

The present invention relates in another embodiment to the use of an antigen, particularly an Aβ peptide antigen or a tau peptide antigen as described in any of the preceding embodiments for inducing a T-cell independent immune response, particularly an Aβ-related or tau-related immune response in an individual, particularly animal or human, particularly an animal or human who is depleted of T-cells, particularly of CD4 T-cells.

The present invention relates in another embodiment to a method for the treatment, alleviation of the symptoms or prevention of disease or condition, particularly an amyloid- or tau-protein associated disease or condition in an individual, particularly an immune tolerant individual or patient or a T-cell activated patient, comprising administering to said individual or patient, particularly animal or human, particularly an animal or human who is depleted of T-cells, particularly of CD4 T-cells, suffering from such a disease or condition, an antigenic construct, particularly an Aβ or tau antigenic construct, as claimed in any of the preceding embodiments, or a T-cell independent antigenic composition comprising said antigenic construct, wherein a T-cell independent immune response, particularly the Aβ or tau immune response, is induced in the treated individual.

In a particular embodiment of the invention, said individual is an elderly human patient depleted of T-cells, particularly of CD4 T-cells,

In another embodiment of the invention, said individual is an immunosuppressed human patient, who is depleted of T-cells, particularly of CD4 T-cells.

In still another embodiment of the invention, said individual is a human patient, who is depleted of T-cells, particularly of CD4 T-cells and suffers from an immunodeficiency disease, particularly from HIV.

In still another embodiment of the invention, said individual is a human patient, who has cancer or another malignancy and is depleted of T-cells, particularly of CD4 T-cells.

In still another embodiment of the invention, said individual is a human patient which exhibits T-cell activation and where a further stimulation of the T-cell response would cause a medical risk.

The present invention relates in another embodiment to a method according to any of the previous embodiments, wherein no adverse inflammatory effect is induced.

The present invention relates in another embodiment to a method according to the previous embodiment, wherein administration of said antigen, particularly of an Aβ or tau-antigenic construct results mainly in the generation of antibodies of non-inflammatory subtypes, particularly of the non-inflammatory Th2 subtype, particularly of isotype IgG1 and IgG2b.

The present invention relates in another embodiment to a method according to the previous embodiments, wherein administration of said Aβ antigenic construct results mainly in the generation of antibodies of the T-cell independent IgG subclass, particularly of the IgG3 isotype.

The present invention relates in another embodiment to a method according to embodiment the previous, embodiments does not lead to a significant increase in inflammation markers in the brain, particularly markers selected from the group consisting of IL-1 β, IL-6, IFN-y and TNF α.

The present invention relates in another embodiment to a method according to any of the preceding embodiments, wherein administration of said Aβ antigenic construct leads to a significant decrease of insoluble, plaque-related- Aβ1-40 and Aβ1-42 in the brain.

The present invention relates in another embodiment to a method according to embodiment (49), wherein administration of said Aβ antigenic construct leads to a significant reduction in the level of soluble Aβ1-42 in the brain.

The present invention relates in another embodiment to a method according to any of the preceding embodiments, wherein the amyloid associated disease or condition is one selected from the group consisting of diseases including, but not limited to, neurological disorders such as Alzheimer's Disease (AD), including diseases or conditions characterized by a loss of cognitive memory capacity such as, for example, mild cognitive impairment (MCI), Lewy body dementia, Down's syndrome, hereditary cerebral hemorrhage with amyloidosis (Dutch type); the Guam Parkinson-Dementia complex; as well as other diseases which are based on or associated with amyloid-like proteins such as progressive supranuclear palsy, multiple sclerosis; Creutzfeld-Jacob disease, Parkinson's disease, HIV-related dementia, ALS (amyotropic lateral sclerosis), Adult Onset Diabetes; senile cardiac amyloidosis; endocrine tumors, stroke, traumatic brain injury, all ocular diseases and others, including macular degeneration and glaucoma, particularly Alzheimer's Disease.

The present invention relates in another embodiment to a method for the treatment, alleviation of the symptoms or prevention of disease or condition, particularly tau-protein associated disease or condition in an individual, particularly an immune tolerant individual or patient, or a T-cell activated patient, according to any of the preceding embodiments, wherein the the tau-associated disease or disorder is a neurodegenerative disorder, particularly a neurodegenerative disease or disorder, which is caused by or associated with the formation of neurofibrillary lesions, particularly a tauopathy, particularly a tauopathy selected from the group consisting of Alzheimer's disease, Creutzfeld-Jakob disease, Dementia pugilistica, Down's Syndrome, Gerstmann-Straussler-Scheinker disease, inclusion-body myositis, and prion protein cerebral amyloid angiopathy, traumatic brain injury, amyotrophic lateral sclerosis/parkinsonism-dementia complex of Guam, Non-Guamanian motor neuron disease with neurofibrillary tangles, argyrophilic grain dementia, corticobasal degeneration, diffuse neurofibrillary tangles with calcification, frontotemporal dementia with parkinsonism linked to chromosome 17, Hallevorden-Spatz disease, multiple system atrophy, Niemann-Pick disease, Type C, Pick's disease, progressive subcortical gliosis, progressive supranuclear palsy, Subacute sclerosing panencephalitis, Tangle only dementia, Postencephalitic Parkinsonism, Myotonic dystrophy, particularly Alzheimer's disease.

Further methods, candidates, compounds and details are disclosed in PCT/EP2006/011861 (published under WO2007/068411) and in EP patent application 09 15 7303.0 filed April 03, 2009, the disclosure of which is incorporated herein by reference in its entirety.

### DESCRIPTION OF THE INVENTION

The present invention also envisages in a particular embodiment that T-cell immunodeficiency diseases can be avoided in an individual, particularly animal or human. Such an individual can be either vaccinated before the onset of the disease or, in case of suffering from a disease or condition, the individual can be treated by the method described herein. In one embodiment, the disease is an amyloid-associated disease. In another embodiment, the disease is a tau-protein associated disease. Such diseases can be, *inter alia*, DiGeorge syndrome, Wiskott-Aldrich syndrome, Ataxia telangiectasia or Chronic mucocutaneous candidiasis.

The supramolecular antigenic constructs according to the present invention or an antigenic composition comprising such an antigenic construct, may be used for the preparation of a vaccine composition for inducing an T-cell independent immune response in an individual, particularly an immune tolerant individual or patient, or a T-cell activated patient, particularly animal or human, particularly an animal or human who is depleted of T-cells, particularly of CD4 T cells. In one embodiment, the supramolecular antigenic construct is used in a method for preventing, treating or alleviating the effects of amyloidosis, a group of diseases and disorders associated with amyloid plaque formation including secondary amyloidosis and age-related amyloidosis including, but not limited to, neurological disorders such as Alzheimer's Disease (AD), including diseases or conditions characterized by a loss of cognitive memory capacity such as, for example, mild cognitive impairment (MCI), Lewy body dementia, Down's syndrome, hereditary cerebral hemorrhage with amyloidosis (Dutch type); the Guam Parkinson-Dementia complex; as well as other diseases which are based on or associated with amyloid-like proteins such as progressive supranuclear palsy, multiple sclerosis; Creutzfeld-Jacob disease, Parkinson's disease, HIV-related dementia, ALS (amyotropic lateral sclerosis), Adult Onset Diabetes; senile cardiac amyloidosis; endocrine tumors, stroke, traumatic brain injury, all ocular diseases and others, including macular degeneration and glaucoma, but particularly a disease or condition characterized by a loss of cognitive memory capacity such as, for example, mild cognitive impairment (MCI) in an individual, particularly an immune tolerant individual or patient, or a T-cell activated patient, particularly animal or human, particularly an animal or human who is depleted of T-cells, particularly of CD4 T-cells comprising administering to said individual a supramolecular antigenic construct according to the present invention, but particularly a vaccine composition comprising such a supramolecular antigenic constructs according to the invention to a an animal, particularly a mammal or a human, affected by such a disorder and thus in need of such a treatment.

In still a further embodiment of the present invention a method is provided for the preparation of a medicament for preventing, treating or alleviating in an individual, particularly an immune tolerant individual or patient, or a T-cell activated patient, particularly animal or human, particularly an animal or human who is depleted of T-cells, particularly of CD4 T-cells, the effects of amyloidosis, a group of diseases and disorders associated with amyloid plaque formation including secondary amyloidosis and age-related amyloidosis including, but not limited to, neurological disorders such as Alzheimer's Disease (AD), including diseases or conditions characterized by a loss of cognitive memory capacity such as, for example, mild cognitive impairment (MCI), Lewy body dementia, Down's syndrome, hereditary cerebral hemorrhage with amyloidosis (Dutch type); the Guam Parkinson-Dementia complex; as well as other diseases which are based on or associated with amyloid-like proteins such as progressive supranuclear palsy, multiple sclerosis; Creutzfeld-Jacob disease, Parkinson's disease, HIV-related dementia, ALS (amyotropic lateral sclerosis), Adult Onset Diabetes; senile cardiac amyloidosis; endocrine tumors, stroke, traumatic brain injury, all ocular diseases and others, including macular degeneration and glaucoma, but particularly a disease or condition characterized by a loss of cognitive memory capacity such as, for example, mild cognitive impairment (MCI), comprising formulating an antibody according to the invention in a pharmaceutically acceptable form.

In a specific embodiment, the present invention makes use of an antigen presentation that results in enhanced exposure and stabilization of a preferred antigen conformation, which ultimately leads to a highly specific immune response and results in the generation of antibodies with unique properties. tau

The present invention provides T-cell independent immunogenic compositions and therapeutic vaccine compositions comprising said immunogenic compositions comprising supramolecular constructs that use antigenic peptides for the treatment, alleviation of the symptoms, delaying onset, or prevention of diseases and disorders, particularly of diseases and disorders which are caused by or associated with amyloid or amyloid-like proteins including amyloidosis, a group of disorders and abnormalities associated with amyloid protein such as Alzheimer's Disease, or diseases and disorders which are caused by or associated with protein tau including tauopathies, in immune tolerant patients with a T-cell deficiency, particularly patients which are depleted of CD4 T-cells. Such antigenic peptides may include, but are not limited to, amyloid or amyloid-like protein such as, for example, prion protein, tau protein, alpha-synuclein, huntingtin, and the like. In one embodiment, the present invention provides immunogenic compositions comprising a supramolecular antigenic construct, particularly an antigenic construct comprising a peptide antigen derived from amyloid protein or amyloid-like protein such as, for example, prion protein, tau protein, alpha-synuclein, huntingtin, and the like, particularly a β-amyloid peptide antigen or a tau peptide antigen according to the invention and as described herein before representative of the N-terminal part of the β-amyloid peptide, which antigenic peptide is modified such that it is capable of maintaining and stabilizing a defined conformation of the antigen, particularly a conformation which is characterized by a balanced proportion of random coil, α-helical and β-sheet portions. This defined conformation leads to the induction of a strong and highly specific immune response, particularly a T-cell independent immune response, upon introduction into an animal or a human.

In particular, the antigenic composition of the invention and as described herein may comprise a conformational antigen, wherein more than 30%, particularly more than 40%, particularly more than 50%, particularly more than 60%, particularly more than 70%, particularly more than 80%, particularly more than 90%, particularly more than 95% and up to 100% is in a beta-sheet conformation.

One way of achieving the formation and stabilization of the desired conformation of the antigenic peptide is by presenting the antigenic peptide attached to, or incorporated or reconstituted, partially or fully, into a carrier such as, for example, a vesicle, a particulate body or molecule or any other means that can suitably serve as a carrier/adjuvant for the antigenic peptide. In a specific embodiment of the invention, the antigenic peptide is attached to, or incorporated or reconstituted in the carrier through weak interactions such as, for example, van der Waal's, hydrophobic or electrostatic interaction, or a combination of two or more of said interactions, such that the peptide is presented with a specific conformation, which is maintained and stabilized by restricting said antigenic peptide in its three dimensional freedom of movement so that conformational changes are prevented or severely restricted.

In a specific embodiment of the invention, the antigenic peptide is presented on the surface of the carrier molecule in a highly repetitive array, particularly a repetitive array comprising at least 10 repetitive antigenic units/carrier molecule, particularly at least 50 repetitive antigenic units/carrier molecule, particularly at least 100 repetitive antigenic units/carrier molecule, particularly at least 200 repetitive antigenic units/carrier molecule, particularly at least 300 repetitive antigenic units/carrier molecule; particularly at least 400 repetitive antigenic units/carrier molecule, particularly at least 500 repetitive antigenic units/carrier molecule.

When a vesicle, a particle or a particulate body is used as a carrier/adjuvant such as, for example, a liposome, the composition of the antigenic peptide may be chosen such that its overall net charge is identical to that of the carrier/adjuvant surface to which the peptide is attached. Electrostatic repulsion forces being effective between the identically charged carrier/adjuvant surface and the antigenic peptide, but particularly the identically charged carrier surface and the amino acid residues constituting the antigenic peptide and more particularly the identically charged carrier surface and the identically charged amino acid residues comprised in the antigenic peptide, may lead to the antigenic peptide taking on a defined, highly specific and stabilized conformation which guarantees a high biological activity. As a result, the antigenic peptide is exposed and presented in a conformation that is highly biologically active in that it allows the immune system of the target organism to freely interact with the antigenic determinants contained in the antigenic construct in the biologically active conformation, which leads to a strong and conformation-specific immune response, resulting in, for example, a high antibody titer in the target organism. By carefully coordinating the overall net charges of the antigenic peptide on the one side and of the carrier to which the peptide becomes attached, incorporated or reconstituted in on the other side, the antigenic peptide is presented exposed on, or in close proximity to, the carrier surface in a conformation that is induced and stabilized by electrostatic repulsion forces being effective between the identically charged carrier surface and the antigenic peptide, but particularly the identically charged carrier surface and the amino acid residues constituting the antigenic peptide and more particularly the identically charged carrier surface and the identically charged amino acid residues comprised in the antigenic peptide. This results in a presentation of the antigenic construct such that is freely accessible to the immune defense machinery of the target organism and thus capable of inducing a strong and highly specific immunogenic response upon administration to an animal or a human. The immunogenic response may be further increased by using a liposome as a carrier, which liposome may function as an adjuvant to increase or stimulate the immune response within the target animal or human to be treated with the therapeutic vaccine according to the invention. Optionally, the liposome may, in addition, contain a further adjuvant such as, for example, lipid A, alum, calcium phosphate, interleukin 1, and/or microcapsules of polysaccharides and proteins, but particularly a detoxified lipid A, such as monophosphoryl or diphosphoryl lipid A, or alum.

In a specific embodiment of the invention an antigenic peptide according to the invention and described herein before, particularly an antigenic peptide the overall net charge of which is negative, is used reconstituted in a liposome, particularly a liposome the constituents of which are chosen such that the net overall charge of the liposome head group is negative. In particular, the liposome is composed of constituents selected from the group consisting of dimyristoyl phosphatidyl choline (DMPC), dimyristoyl phosphatidyl ethanolamine (DMPEA), dimyristoyl phosphatidyl glycerol (DMPG) and cholesterol and, optionally, further contains monophosphoryl lipid A or any other adjuvant that can be suitably used within the scope of the present invention such as, for example, alum, calcium phosphate, interleukin 1, and/or microcapsules of polysaccharides and proteins.

In another specific embodiment of the invention, a modified peptide antigen according to the invention and as described herein before is provided covalently bound to an anchor-type molecule which is capable of inserting into the carrier/adjuvant thereby fixing the peptide to the carrier/adjuvant and presenting it on or in close proximity to the surface of a carrier/adjuvant molecule such that electrostatic forces can become effective as described herein before.

When liposomes are used as a carrier/adjuvant, the antigenic peptide construct generally has a hydrophobic tail that inserts into the liposome membrane as it is formed. Additionally, antigenic peptides can be modified to contain a hydrophobic tail so that it can be inserted into the liposome.

The supramolecular antigenic constructs of the present invention generally comprise peptides modified to enhance antigenic effect wherein such peptides may be modified via pegylation (using polyethylene glycol or modified polyethylene glycol), or modified via other methods such by palmitic acid as described herein before, poly-amino acids (eg poly-glycine, poly-histidine), poly-saccharides (eg polygalacturonic acid, polylactic acid, polyglycolide, chitin, chitosan), synthetic polymers (polyamides, polyurethanes, polyesters) or co-polymers (eg poly(methacrylic acid) and N-(2-hydroxy) propyl methacrylamide) and the like.

In a specific embodiment of the invention, antigenic peptides according to the invention and as described herein before are provided, which are modified to contain a hydrophobic tail so that said peptides can be inserted into the liposome. In particular, the β-amyloid peptide may be modified by a lipophilic or hydrophobic moiety that facilitates insertion into the lipid bilayer of the carrier/adjuvant. The lipophilic or hydrophobic moieties of the present invention may be fatty acids, triglycerides and phospholipids, particularly fatty acids, triglycerides and phospholipids, wherein the fatty acid carbon back bone has at least 10 carbon atoms particularly lipophilic moieties having fatty acids with a carbon backbone of at least approximately 14 carbon atoms and up to approximately 24 carbon atoms, more particularly hydrophobic moieties having a carbon backbone of at least 14 carbon atoms. Examples of hydrophobic moieties include, but are not limited to, palmitic acid, stearic acid, myristic acid, lauric acid, oleic acid, linoleic acid, linolenic acid and cholesterol or DSPE. In a specific embodiment of the invention the hydrophobic moiety is palmitic acid.

Palmitoylation, while providing an anchor for the peptide in the liposome bilayer, due to the relative reduced length of the C_{16:0} fatty acid moiety leads to the peptide being presented exposed on or in close proximity to the liposome surface. Therefore, the cells processing the antigen will have to take up the entire liposome with the peptide.

In another embodiment of the invention, PEG is used in the preparation of a supramolecular construct, wherein the free PEG terminus is covalently attached to a molecule of phosphatidylethanolamine (where the fatty acid can be: myristic, palmitic, stearic, oleic etc. or combination thereof). This supramolecular structure may be reconstituted in liposomes consisting of phospholipids and cholesterol (phosphatidylethanol amine, phosphatidyl glycerol, cholesterol in varied molar ratios. Other phospholipids can be used. Lipid A is used at a concentration of approximately 40 µg/pmole of phospholipids.

In certain embodiments, the supramolecular antigenic constructs of the present invention comprise an antigenic peptide sequence as described herein before, covalently attached to pegylated lysine- at least one at each terminus but particularly 1 or 2 at each terminus. The length of the PEG (polyethylenglycol) chain may vary from n = 8 to n = 150.000 or more, particularly from n = 10 to n = 80.000, more particularly from n = 10 to n = 10.000. In a specific embodiment of the invention the length of the PEG chain is not more than n = 45, particularly between n = 5 and n = 40, more particularly between n = 10 and n = 30, and even more particularly n = 10. Liposomes that can be used in the compositions of the present invention include those known to one skilled in the art. Any of the standard lipids useful for making liposomes may be used. Standard bilayer and multi-layer liposomes may be used to make compositions of the present invention. While any method of making liposomes known to one skilled in the art may be used, the most preferred liposomes are made according to the method of Alving et al., Infect. Immun. 60:2438-2444, 1992, hereby incorporated by reference. The liposome can optionally contain an adjuvant or and immunomodulator or both. A preferred immunomodulator is lipid A, particularly a detoxified lipid A such as, for example, monophosphoryl or diphosphoryl lipid A.

The liposome may have a dual function in that it can be used as a carrier comprising the supramolecular construct as described herein before and, at the same time, function as an adjuvant to increase or stimulate the immune response within the target animal or human to be treated with the therapeutic vaccine according to the invention. Optionally, the liposome may, in addition, contain a further adjuvant or and immunomodulator or both such as, for example, lipid A, alum, calcium phosphate, interleukin 1, and/or microcapsules of polysaccharides and proteins, but particularly a lipid A, more particularly a detoxified lipid A, such as monophosphoryl or diphosphoryl lipid A, or alum.

The immunogenic composition and/or the therapeutic vaccine of the present invention comprising a supramolecular antigenic construct according to the invention and as described herein before may be prepared in the form of a liquid solution, or of an injectable suspension, or else in a solid form suitable for solubilization prior to injection in the context of, for example, a kit for making use of the present composition, as described below.

The immunogenic composition and/or the therapeutic vaccine of the present invention comprising a supramolecular antigenic construct is administered to a human or animal, particularly to a human or animal suffering from an amyloid- or tau-associated disease to induce an immune response in said human or animal to alleviate symptoms associated with the disease or to restore a condition found in healthy individuals which are unaffected by the disease.

The immunogenic composition and/or the therapeutic vaccine of the present invention are administered to a human or animal by any appropriate standard routes of administration. In general, the composition may be administered by topical, oral, rectal, nasal or parenteral (for example, intravenous, subcutaneous, or intramuscular) routes. In addition, the composition may be incorporated into sustained release matrices such as biodegradable polymers, the polymers being implanted in the vicinity of where delivery is desired, for example, at the site of a tumor. The method includes administration of a single dose, administration of repeated doses at predetermined time intervals, and sustained administration for a predetermined period of time.

In particular, the antigenic peptide composition according to the invention is administered by parenteral, particularly by intra-peritoneal, intraveneous, subcutaneous and intra-muscular injection.

The dosage of the composition will depend on the condition being treated, the particular composition used, and other clinical factors such as weight, size and condition of the patient, body surface area, the particular compound or composition to be administered, other drugs being administered concurrently, and the route of administration.

The immunogenic composition and/or the therapeutic vaccine according to the invention may be administered in combination with other biologically active substances and procedures for the treatment of diseases. The other biologically active substances may be part of the same composition already comprising the immunogenic composition and/or the therapeutic vaccine according to the invention, in form of a mixture, wherein the therapeutic vaccine and the other biologically active substance are intermixed in or with the same pharmaceutically acceptable solvent and/or carrier or may be provided separately as part of a separate compositions, which may be offered separately or together in form a kit of parts.

The immunogenic composition and/or the therapeutic vaccine according to the invention may be administered concomitantly with the other biologically active substance or substances, intermittently or sequentially. For example, the immunogenic composition and/or the therapeutic vaccine according to the invention may be administered simultaneously with a first additional biologically active substance or sequentially after or before administration of said composition. If an application scheme is chosen where more than one additional biologically active substance are administered together with the at least one immunogenic composition and/or therapeutic vaccine according to the invention, the compounds or substances may partially be administered simultaneously, partially sequentially in various combinations.

It is another object of the present invention to provide for mixtures of an immunogenic composition and/or a therapeutic vaccine according to the invention and, optionally, one or more further biologically active substances, as well as to methods of using such a composition according to the invention, or mixtures thereof for the prevention and/or therapeutic treatment and/or alleviation of the effects of amyloidoses, a group of diseases and disorders associated with amyloid plaque formation including secondary amyloidoses and age-related amyloidoses, or tauopathies a tau-associated neurodegenerative disease or disorder which is caused by or associated with the formation of neurofibrillary lesions, as disclosed herein before, in immune tolerant patients or a T-cell activated patients, particularly in patients with a T-cell deficiency, particularly patients which are depleted of CD4 T-cells.

The mixtures according to the invention may comprise, in addition to an immunogenic composition and/or a therapeutic vaccine according to the invention, a biologically active substance such as, for example, known compounds used in the medication of amyloidoses, a group of diseases and disorders associated with amyloid or amyloid-like protein such as the Aβ protein involved in Alzheimer's Disease including an antibody raised against an amyloidogenic peptide antigen, particularly an antibody raised against an amyloidogenic antigen presented in form of a supramolecular antigenic construct, more particularly an antibody according to the present invention and as disclosed herein.

In another embodiment of the invention, the other biologically active substance or compound may also be a therapeutic agent that may be used in the treatment of diseases and disorders which are caused by or associated with amyloid or amyloid-like proteins including amyloidosis caused by amyloid β, or tau-associated neurodegenerative diseases or disorders including tauopathies, or may be used in the medication of other neurological disorders. The other biologically active substance or compound may exert its biological effect by the same or a similar mechanism as the immunogenic composition and/or the therapeutic vaccine according to the invention or by an unrelated mechanism of action or by a multiplicity of related and/or unrelated mechanisms of action.

Generally, the other biologically active compound may include neutron-transmission enhancers, psychotherapeutic drugs, acetylcholine esterase inhibitors, calcium-channel blockers, biogenic amines, benzodiazepine tranquillizers, acetylcholine synthesis, storage or release enhancers, acetylcholine postsynaptic receptor agonists, monoamine oxidase-A or -B inhibitors, N-methyl-D-aspartate glutamate receptor antagonists, non-steroidal anti-inflammatory drugs, antioxidants, and serotonergic receptor antagonists. In particular, the mixture according to the invention may comprise at least one other biologically active compound selected from the group consisting of compounds against oxidative stress, anti-apoptotic compounds, metal chelators, inhibitors of DNA repair such as pirenzepin and metabolites, 3-amino-1-propanesulfonic acid (3APS), 1,3-propanedisulfonate (1,3PDS), secretase activators, β- and y -secretase inhibitors, tau proteins, neurotransmitter, β-sheet breakers, anti-inflammatory molecules, or cholinesterase inhibitors (ChEls) such as tacrine, rivastigmine, donepezil, and/or galantamine and other drugs and nutritive supplements, together with an therapeutic vaccine according to the invention and, optionally, a pharmaceutically acceptable carrier and/or a diluent and/or an excipient.

In a further embodiment, the mixtures according to the invention may comprise niacin or memantine together with an immunogenic composition and/or a therapeutic vaccine according to the invention and, optionally, a pharmaceutically acceptable carrier and/or a diluent and/or an excipient.

In still another embodiment of the invention mixtures are provided that comprise "atypical antipsychotics" such as, for example clozapine, ziprasidone, risperidone, aripiprazole or olanzapine for the treatment of positive and negative psychotic symptoms including hallucinations, delusions, thought disorders (manifested by marked incoherence, derailment, tangentiality), and bizarre or disorganized behavior, as well as anhedonia, flattened affect, apathy, and social withdrawal, together with an immunogenic composition and/or a therapeutic vaccine according to the invention and, optionally, a pharmaceutically acceptable carrier and/or a diluent and/or an excipient for the treatment, alleviation of the symptoms or prevention of diseases and disorders which are caused by or associated with amyloid or amyloid-like proteins including amyloidosis, a group of disorders and abnormalities associated with amyloid protein such as Alzheimer's Disease, or of diseases and disorders which are caused by or associated with tau protein, such as tau-associated neurodegenerative diseases or disorders which are caused by or associated with the formation of neurofibrillary lesions including tauopathies, in immune tolerant patients or a T-cell activated patients, particularly in patients with a T-cell deficiency, particularly patients which are depleted of CD4 T-cells.

In a specific embodiment of the invention, the compositions and mixtures according to the invention and as described herein before comprise immunogenic composition according to the invention and the biologically active substance, respectively, in a therapeutically or prophylactically effective amount.

Other compounds that can be suitably used in mixtures in combination with the immunogenic composition and/or the therapeutic vaccine according to the invention are described, for example, in WO 2004/058258 (see especially pages 16 and 17) including therapeutic drug targets (page 36-39), alkanesulfonic acids and alkanolsulfuric acid (pages 39-51), cholinesterase inhibitors (pages 51-56), NMDA receptor antagonists (pages 56-58), estrogens (pages 58-59), non-steroidal anti-inflammatory drugs (pages 60-61), antioxidants (pages 61-62), peroxisome proliferators-activated receptors (PPAR) agonists (pages 63-67), cholesterol-lowering agents (pages 68-75); amyloid inhibitors (pages 75-77), amyloid formation inhibitors (pages 77-78), metal chelators (pages 78-79), anti-psychotics and anti-depressants (pages 80-82), nutritional supplements (pages 83-89) and compounds increasing the availability of biologically active substances in the brain (see pages 89-93) and prodrugs (pages 93 and 94 ), which document is incorporated herein by reference, but especially the compounds mentioned on the pages indicated above.

It is long known that vaccination of an animal or human host with a normal host protein may lead to the development of auto-antibodies directed against the host protein resulting in disorders collectively known as autoimmune disorders. Aβ and its APP precursor protein are such normal proteins. Using these host proteins in a vaccination thus has the potential of creating undesired side-effects. There is some evidence in the literature that Aβ may activate a neuroinflammatory response which may partly be caused by an overactivation of the complement system, which is already highly activated in patients suffering from Alzheimer's Disease or other neurodegenerative diseases.

Human Aβ in its β-sheet conformation is a powerful activator of the human complement system. It strongly binds to the collagen tail of the human complement C1q. Overactivation of the complement system can result in the host's natural defense system turning around and leading to autodestruction of cells and tissues including neurons and their processes. For example, the membrane attack complex (MAC) which is part of the host's natural defense system and protects the host against invading bacteria and viruses by inserting itself into said bacteria and viruses, upon overactivation can insert itself into host cells and cause autodistruction. Overactivation may further lead to the stimulation of microglia to produce toxic compounds such as oxygen-free radicals and harmful proteases.

It is thus a further object of the present invention to prevent potential side effects such as neurological complications caused by vaccinating an animal or a human suffering from an autoimmune disease with an autoantigen, which has the potential to further stimulate an already over-activated complement system. This can be achieved within the scope of the present invention by administering an Aβ peptide antigen, particularly a palmitoylated Aβ peptide antigen, more particularly the palmitoylated Aβ₁₋₁₅ peptide antigen, but especially the palmitoylated Aβ₁₋₁₅ peptide antigen (ACI-24, Aβ₁₋₁₅) in combination with a complement inhibitor.

It is thus another embodiment of the invention to provide a vaccine composition comprising in addition to an Aβ peptide antigen, particularly the Aβ peptide antigen according to the invention and described herein before; an inhibitor of the complement system.

The complement inhibitor may be a compound selected from the group consisting of soluble human complement Receptor 1, anti- human complement protein C5 such as, for example, a humanized anti C5 monoclonal antibody or a single-chain fragment of a humanized monoclonal antibody, C1-esterase inhibitor-N and Natural human C1 Inhibitor.

The modified amyloid peptide antigen such as, for example, the amyloid beta 1-15 peptide may be synthesized following the method reported in Nicolau et. al. (2002) Proc Natl. Acad. Sci USA 99, 2332-2337. The approach reported in Nicolau et al. may be modified by first synthesizing the antigenic peptide which is then further modified by an on-resin grafting of a lipophilic or hydrophobic moiety, to the terminal amino acid residues of the pre-formed peptide. In particular, a protected amino acid, particularly a Fmoc-protected amino acid, is attached to a resin using known coupling chemistry. The protecting group is removed and a second protected amino acid residue coupled. Standard automated peptide synthesis using known protection chemistry, particularly Fmoc/tBu chemistry, and standard side-chain protecting groups are then used to synthesis the Aβ antigenic peptide, particularly the Aβ₁₋₁₅ antigenic peptide by coupling on amino acids 1 to 15 of amyloid protein Aβ₁₋₄₂.to produce the peptide fragment with a given sequence. In a final step two further protected amino acids are coupled to the growing peptide fragment. The Mtt groups can then be selectively cleaved and coupled to palmitic acid. After washing of the resin, the protecting group is removed and the resin simultaneously cleaved, followed by side-chain deprotections using standard methodology. The final product can then be obtained in high purity and its identity confirmed by methods known in the art such as, for example, electrospray mass spectrometry.

The lipophilic or hydrophobic moiety according to the present invention may be a fatty acid, a triglyceride or a phospholipid wherein the fatty acid carbon back bone has at least 10 carbon atoms. Particularly, the lipophilic or hydrophobic moiety is a fatty acid with a carbon backbone of at least approximately 14 carbon atoms and up to approximately 24 carbon atoms, with each individual number of carbon atom falling within this range also being part of the present invention. More particularly, the lipophilic or hydrophobic moiety has a carbon backbone of at least 14 carbon atoms, but especially 16 carbon atoms. Examples of hydrophobic moieties include, but are not limited to, palmitic acid, stearic acid, myristic acid, lauric acid, oleic acid, linoleic acid, and linolenic acid. In a specific embodiment of the present invention the lipophilic or hydrophobic moiety is palmitic acid.

Liposomal antigens according to the invention may then be prepared as described in Nicolau et al., 2002. The modified amyloid Aβ antigenic peptide, particularly the modified Aβ₁₋₁₅ antigenic peptide may be reconstituted in a construct consisting of liposomes, particularly liposomes made of dimyristoyl phosphatidyl choline (DMPC), dimyristoyl phosphatidyl ethanolamine (DMPEA), dimyristoyl phosphatidyl glycerol (DMPG) and cholesterol, optionally containing monophosphoryl lipid A.

In a specific embodiment of the invention liposomes with lipid A are used as adjuvant to prepare the anti-amyloid vaccine. Dimyristoylphosphatidyl-choline, -glycerol and cholesterol are mixed, particularly in a molar ratio of 0.9:1.0:0.7. A strong immunmodulator such as, for example, monophosphoryl lipid A is then added at a suitable concentration, particularly at a concentration of between 30 and 50 mg per mmol, more particularly at 40 mg per mmol of phospholipids. The modified antigenic Aβ peptide is then added at a molar ratio peptide to phospholipids of between 1:30 and 1:200, particularly at a molar ratio of between 1:50 and 1:120, more particularly of 1:100. Solvents are removed, for example through evaporation, and the resulting film hydrated with sterile buffer solution such as, for example PBS.

Liposomes may also be prepared by the crossflow injection technique as described, for example, in Wagner et al. (2002) Journal of Liposome Research Vol 12(3), pp 259 - 270. During the injection of lipid solutions into an aqueous buffer system, lipids tend to form "precipitates", followed by self arrangement in vesicles. The obtained vesicle size depends on factors such as lipid concentration, stirring rate, injection rate, and the choice of lipids. The preparation system may consist of a crossflow injection module, vessels for the polar phase (e.g. a PBS buffer solution), an ethanol/lipid solution vessel and a pressure device, but particularly a nitrogen pressure device. While the aqueous or polar solution is pumped through the crossflow injection module the ethanol/lipid solution is injected into the polar phase with varying pressures applied.

For determining immuogenicity of the modified Aβ antigenic construct a suitable animal selected from the group consisting of mice, rats, rabbits, pigs, birds, etc., but particularly mice, especially C57BL/6 mice are immunized with the antigenic peptide. Immunogenicity of the antigenic construct is determined by probing Sera samples in suitable time intervals after immunization using an immunoassay such as, for example, an ELISA assay:
The modified antigenic construct, particularly the palmitoylated antigenic construct and, more particularly, the palmitoylated Aβ₁₋₁₅ construct or the palmitoylated Tau5-20 [pY18] is used for the immunization of an immune tolerant animal or human patient or a T-cell activated patient, suffering from symptoms associated with amyloidosis, a group of diseases and disorders associated with amyloid plaque formation including secondary amyloidosis and age-related amyloidosis or any other amyloid-associated disease, or from symptoms associated with tauopathies, a group of diseases and disorders which are caused by or associated with tau protein and caused by or associated with the formation of neurofibrillary lesions, wherein said animal, particularly said mammal or human, has a T-cell deficiency, particularly a CD4 T-cell deficiency.

The supramolecular antigenic construct according to the present invention, but particularly an immunogenic composition and/or a therapeutic vaccine comprising such a supramolecular antigenic construct according to the invention, is administered to an animal, particularly a mammal or a human, by any appropriate standard routes of administration. In general, the composition may be administered by topical, oral, rectal, nasal or parenteral (for example, intravenous, subcutaneous, or intramuscular) routes, wherein said animal, particularly said mammal or human, has a T-cell deficiency, particularly a CD4 T-cell deficiency. In addition, the composition may be incorporated into sustained release matrices such as biodegradable polymers, the polymers being implanted in the vicinity of where delivery is desired, for example, at the site of a tumor. The method includes administration of a single dose, administration of repeated doses at predetermined time intervals, and sustained administration for a predetermined period of time.

In a specific embodiment of the invention the antigenic construct according to the invention, particularly an immunogenic composition and/or a therapeutic vaccine comprising said antigenic construct in a pharmaceutically acceptable form, is administered in repeated doses, in particular in 1 to 15 doses, more particularly in 2 to 10 doses, more particularly in 3 to 7 doses and even more particularly in 4 to 6 doses, in time intervals of between 1 and 10 weeks, particularly in time intervals of between 1 and 6 weeks, more particularly in time intervals of between 1 and 4 weeks, and even more particularly in time intervals of between 2 and 3 weeks. The immune response is monitored by taking Sera samples at a suitable time after boosting, particularly 3 to 10 days after boosting, more particularly 4 to 8 days after boosting and more particularly 5 to 6 days after boosting and determining the immunogenicity of the antigenic construct using known methodology, particularly one of the commonly used immunoassays such as, for example, an ELISA assay. Immunization with the antigenic construct according to the invention, but particularly with an immunogenic composition and/or a therapeutic vaccine comprising the antigenic construct according to the invention in a pharmaceutically acceptable form leads to a significant and highly specific T-cell independent immune response in the treated animal or human.

The supramolecular antigenic construct compositions of the present invention are administered to an immune tolerant human or animal patient or a T-cell activated patients, particularly a human or an animal who is substantially depleted of T-cells, particularly of CD4 T-cells, to induce immunity to antigenic agents such as infectious organisms or to antigenic aspects of other pathological conditions such as β-amyloid aggregation (Alzheimer's Disease) or hyper proliferative disorders such as cancer. The immunized human or animal develops circulating antibodies against the infectious organism, thereby reducing or inactivating its ability to stimulate disease.

The compositions of the present invention are administered to a human or animal by any appropriate means, preferably by injection. For example, a modified antigenic peptide reconstituted in liposomes is administered by subcutaneous injection. Whether internally produced or provided from external sources, the circulating antibodies bind to antigen and reduce or inactivate its ability to stimulate disease.

In certain embodiments, the supramolecular antigenic constructs comprise a peptide having the amino acid sequence of β-amyloid. The peptides may also comprise or correspond to whole amyloid beta peptide and active fragments thereof. Additionally, peptides useful for the present invention further comprise Aβ.

### DEFINITIONS

The terms "individual, particularly animal or human, is T-cell deficient", "T-cell deficient", "substantially T-cell deficient" or "T-cell deficiency", as used herein, refer to an individual substantially lacking functional T-cell(s).

The terms "polypeptide", "peptide", and "protein", as used herein, are interchangeable and are defined to mean a biomolecule composed of amino acids linked by a peptide bond.

The term "peptides," are chains of amino acids (typically L-amino acids) whose alpha carbons are linked through peptide bonds formed by a condensation reaction between the carboxyl group of the alpha carbon of one amino acid and the amino group of the alpha carbon of another amino acid. The terminal amino acid at one end of the chain (i.e., the amino terminal) has a free amino group, while the terminal amino acid at the other end of the chain (i.e., the carboxy terminal) has a free carboxyl group. As such, the term "amino terminus" (abbreviated N-terminus) refers to the free alpha-amino group on the amino acid at the amino terminal of the peptide, or to the alpha-amino group (imino group when participating in a peptide bond) of an amino acid at any other location within the peptide. Similarly, the term "carboxy terminus" (abbreviated C-terminus) refers to the free carboxyl group on the amino acid at the carboxy terminus of a peptide, or to the carboxyl group of an amino acid at any other location within the peptide.

Typically, the amino acids making up a peptide are numbered in order, starting at the amino terminal and increasing in the direction toward the carboxy terminal of the peptide. Thus, when one amino acid is said to "follow" another, that amino acid is positioned closer to the carboxy terminal of the peptide than the preceding amino acid.

The term "residue" is used herein to refer to an amino acid that is incorporated into a peptide by an amide bond. As such, the amino acid may be a naturally occurring amino acid or, unless otherwise limited, may encompass known analogs of natural amino acids that function in a manner similar to the naturally occurring amino acids (i.e., amino acid mimetics). Moreover, an amide bond mimetic includes peptide backbone modifications well known to those skilled in the art.

The phrase "consisting essentially of" is used herein to exclude any elements that would substantially alter the essential properties of the peptides to which the phrase refers. Thus, the description of a peptide "consisting essentially of ..." excludes any amino acid substitutions, additions, or deletions that would substantially alter the biological activity of that peptide.

Furthermore, one of skill will recognize that, as mentioned above, individual substitutions, deletions or additions which alter, add or delete a single amino acid or a small percentage of amino acids (typically less than 5%, more typically less than 1%) in an encoded sequence are conservatively modified variations where the alterations result in the substitution of an amino acid with a chemically similar amino acid. Conservative substitution tables providing functionally similar amino acids are well known in the art. The following six groups each contain amino acids that are conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).

The phrases "isolated" or "biologically pure" refer to material which is substantially or essentially free from components which normally accompany it as found in its native state. Thus, the peptides described herein do not contain materials normally associated with their in situ environment. Typically, the isolated, immunogenic peptides described herein are at least about 80% pure, usually at least about 90%, and preferably at least about 95% as measured by band intensity on a silver stained gel.

Protein purity or homogeneity may be indicated by a number of methods well known in the art, such as polyacrylamide gel electrophoresis of a protein sample, followed by visualization upon staining. For certain purposes high resolution will be needed and HPLC or a similar means for purification utilized. When the immunogenic peptides are relatively short in length (i.e., less than about 50 amino acids), they are often synthesized using standard chemical peptide synthesis techniques.

Solid phase synthesis in which the C-terminal amino acid of the sequence is attached to an insoluble support followed by sequential addition of the remaining amino acids in the sequence is a preferred method for the chemical synthesis of the immunogenic peptides described herein. Techniques for solid phase synthesis are known to those skilled in the art.

Alternatively, the immunogenic peptides described herein are synthesized using recombinant nucleic acid methodology. Generally, this involves creating a nucleic acid sequence that encodes the peptide, placing the nucleic acid in an expression cassette under the control of a particular promoter, expressing the peptide in a host, isolating the expressed peptide or polypeptide and, if required, renaturing the peptide. Techniques sufficient to guide one of skill through such procedures are found in the literature.

Once expressed, recombinant peptides can be purified according to standard procedures, including ammonium sulfate precipitation, affinity columns, column chromatography, gel electrophoresis and the like. Substantially pure compositions of about 50% to 95% homogeneity are preferred, and 80% to 95% or greater homogeneity are most preferred for use as therapeutic agents. One of skill in the art will recognize that after chemical synthesis, biological expression or purification, the immunogenic peptides may possess a conformation substantially different than the native conformations of the constituent peptides. In this case, it is often necessary to denature and reduce the antiproliferative peptide and then to cause the peptide to re-fold into the preferred conformation. Methods of reducing and denaturing proteins and inducing re-folding are well known to those of skill in the art.

Antigenicity of the purified protein may be confirmed, for example, by demonstrating reaction with immune serum, or with antisera produced against the protein itself.

The terms "a", "an" and "the" as used herein are defined to mean "one or more" and include the plural unless the context is inappropriate.

The terms "detecting" or "detected" as used herein mean using known techniques for detection of biologic molecules such as immunochemical or histological methods and refer to qualitatively or quantitatively determining the presence or concentration of the biomolecule under investigation.

By "isolated" is meant a biological molecule free from at least some of the components with which it naturally occurs.

The terms "antibody" or "antibodies" as used herein is an art recognized term and is understood to refer to molecules or active fragments of molecules that bind to known antigens, particularly to immunoglobulin molecules and to immunologically active portions of immunoglobulin molecules, i.e. molecules that contain a binding site that immunospecifically binds an antigen. The immunoglobulin according to the invention can be of any type (IgG, IgM, IgD, IgE, IgA and IgY) or class (lgG1, IgG2, IgG3, IgG4, IgA1 and IgA2) or subclasses of immunoglobulin molecule.

"Antibodies" are intended within the scope of the present invention to include monoclonal antibodies, polyclonal, chimeric, single chain, bispecific, simianized, human and humanized antibodies as well as active fragments thereof. Examples of active fragments of molecules that bind to known antigens include Fab and F(ab')₂ fragments, including the products of a Fab immunoglobulin expression library and epitope-binding fragments of any of the antibodies and fragments mentioned above.

These active fragments can be derived from an antibody of the present invention by a number of techniques. For example, purified monoclonal antibodies can be cleaved with an enzyme, such as pepsin, and subjected to HPLC gel filtration. The appropriate fraction containing Fab fragments can then be collected and concentrated by membrane filtration and the like. For further description of general techniques for the isolation of active fragments of antibodies, see for example, Khaw, B. A. et al. J. Nucl. Med. 23:1011-1019 (1982); Rousseaux et al. Methods Enzymology, 121:663-69, Academic Press, 1986.

A "humanized antibody" refers to a type of engineered antibody having its CDRs derived from a non-human donor immunoglobulin, the remaining immunoglobulin-derived parts of the molecule being derived from one (or more) human immunoglobulin(s). In addition, framework support residues may be altered to preserve binding affinity. Methods to obtain "humanized antibodies" are well known to those skilled in the art. (see, e.g., Queen et al., Proc. Natl Acad Sci USA, 86:10029-10032 (1989), Hodgson et al., Bio/Technoloy, 9:421 (1991)).

A "humanized antibody" may also be obtained by a novel genetic engineering approach that enables production of affinitiy-matured humanlike polyclonal antibodies in large animals such as, for example, rabbits (http://www.rctech.com/bioventures/therapeutic.php).

The term "monoclonal antibody" is also well recognized in the art and refers to an antibody that is mass produced in the laboratory from a single clone and that recognizes only one antigen. Monoclonal antibodies are typically made by fusing a normally short-lived, antibody-producing B cell to a fast-growing cell, such as a cancer cell (sometimes referred to as an "immortal" cell). The resulting hybrid cell, or hybridoma, multiplies rapidly, creating a clone that produces large quantities of the antibody.

"Functionally equivalent antibody" is understood within the scope of the present invention to refer to an antibody which substantially shares at least one major functional property with an antibody mentioned above and herein described comprising: binding specificity to the β-amyloid protein, particularly to the Aβ₁₋₄₂ protein, and more particularly to the 4-16 epitopic region of the Aβ₁₋₄₂ protein, immunoreactivity *in vitro,* inhibition of aggregation of the Aβ₁₋₄₂ monomers into high molecular polymeric fibrils and/or disaggregation of preformed Aβ₁₋₄₂ polymeric fibrils, and/or a β-sheet breaking property and alleviating the effects of disorders associated with amyloidosis, a group of diseases and disorders associated with amyloid plaque formation including secondary amyloidosis and age-related amyloidosis including, but not limited to, neurological disorders such as Alzheimer's Disease (AD), including diseases or conditions characterized by a loss of cognitive memory capacity such as, for example, mild cognitive impairment (MCI), Lewy body dementia, Down's syndrome, hereditary cerebral hemorrhage with amyloidosis (Dutch type); the Guam Parkinson-Dementia complex; as well as other diseases which are based on or associated with amyloid-like proteins such as progressive supranuclear palsy, multiple sclerosis; Creutzfeld-Jacob disease, Parkinson's disease, HIV-related dementia, ALS (amyotropic lateral sclerosis), Adult Onset Diabetes; senile cardiac amyloidosis; endocrine tumors, stroke, traumatic brain injury, all ocular diseases and others, including macular degeneration and glaucoma, when administered prophylactically or therapeutically. The antibodies can be of any class such as IgG, IgM, or IgA, etc or any subclass such as IgG1, IgG2a, etc and other subclasses mentioned herein above or known in the art. Further, the antibodies can be produced by any method, such as phage display, or produced in any organism or cell line, including bacteria, insect, mammal or other type of cell or cell line which produces antibodies with desired characteristics, such as humanized antibodies. The antibodies can also be formed by combining a Fab portion and an Fc region from different species.

The term "antigen" refers to an entity or fragment thereof which can induce an immune response in an organism, particularly an animal, more particularly a mammal including a human. The term includes immunogens and regions responsible for antigenicity or antigenic determinants.

As used herein, the term "soluble" means partially or completely dissolved in an aqueous solution.

Also as used herein, the term "immunogenic" refers to substances which elicit or enhance the production of antibodies, T-cells and other reactive immune cells directed against an immunogenic agent and contribute to an immune response in humans or animals.

An immune response occurs when an individual produces sufficient antibodies, T-cells and other reactive immune cells against administered immunogenic compositions of the present invention to moderate or alleviate the disorder to be treated.

The term "hybridoma" is art recognized and is understood by those of ordinary skill in the art to refer to a cell produced by the fusion of an antibody-producing cell and an immortal cell, e.g. a multiple myeloma cell. This hybrid cell is capable of producing a continuous supply of antibody. See the definition of "monoclonal antibody" above and the Examples below for a more detailed description of the method of fusion.

The term "carrier" as used herein means a structure in which antigenic peptide or supramolecular construct can be incorporated into or can be associated with, thereby presenting or exposing antigenic peptides or part of the peptide to the immune system of a human or animal. Any particle that can be suitably used in animal or human therapy such as, for example, a vesicle, a particle or a particulate body may be used as a carrier within the context of the present invention.

The term "carrier" further comprises methods of delivery wherein supramolecular antigenic construct compositions comprising the antigenic peptide may be transported to desired sites by delivery mechanisms. One example of such a delivery system utilizes colloidal metals such as colloidal gold.

Carrier proteins that can be used in the supramolecular antigenic construct compositions of the present invention include, but are not limited to, maltose binding protein "MBP"; bovine serum albumin "BSA"; keyhole lympet hemocyanin "KLH"; ovalbumin; flagellin; thyroglobulin; serum albumin of any species; gamma globulin of any species; syngeneic cells; syngeneic cells bearing Ia antigens; and polymers of D- and/or L- amino acids.

In the supramolecular antigenic construct according to the present invention, the liposome may have a dual function in that it can be used as a carrier comprising the supramolecular construct as described herein before and, at the same time, function as an adjuvant to increase or stimulate the immune response within the target animal or human to be treated with the therapeutic vaccine according to the invention. It is also to be understood that the supramolecular antigenic construct compositions of the present invention can further comprise additional adjuvants including, but not limited to, keyhole limpet hemocyanin (KLH), bovine serum albumin (BSA) and other adjuvants such as, for example, lipid A, alum, calcium phosphate, interleukin 1, and/or microcapsules of polysaccharides and proteins, but particularly a detoxified lipid A, such as monophosphoryl or diphosphoryl lipid A, or alum, further preservatives, diluents, emulsifiers, stabilizers, and other components that are known and used in vaccines of the prior art. Moreover, any adjuvant system known in the art can be used in the composition of the present invention. Such adjuvants include, but are not limited to, Freund's incomplete adjuvant, Freund's complete adjuvant, polydispersed β-(1,4) linked acetylated mannan ("Acemannan"), T_{ITERMAX}^{®} (polyoxyethylene-polyoxypropylene copolymer adjuvants from CytRx Corporation), modified lipid adjuvants from Chiron Corporation, saponin derivative adjuvants from Cambridge Biotech, killed *Bordetella pertussis*, the lipopolysaccharide (LPS) of gram-negative bacteria, large polymeric anions such as dextran sulfate, and inorganic gels such as alum, aluminum hydroxide, or aluminum phosphate.

Further, the term "effective amount" refers to the amount of antigenic/immunogenic composition which, when administered to a human or animal, elicits an immune response. The effective amount is readily determined by one of skill in the art following routine procedures.

An "immune tolerant patient" as used herein refers to an animal or human patient which shows a limited ability to respond to antigens, particularly non-self antigens, but especially new antigens such as, for examples, new antigens present in newly emerging diseases. This limitation may be due, at least in part, to the chronological age of CD4+ T-cells. Further, an "immune tolerant patient" may exhibit an impaired longterm CD4+ T-cell immune response to antigen exposure due to defects in the proliferation and cytokine secretion of memory T cells during recall responses.

A "T-cell activated patient" as used herein refers to an animal or human patient which exhibits T-cell activation and where a further stimulation of the T-cell response would cause a medical risk.

An "immunocompromised patient" as used herein refers to an animal or human patient having an immune system that has been impaired by age, disease such as HIV, or cancer, or by treatment such as, for example, treatment against inflammatory diseases including, but not limited to, Rheumatoid Arthritis, Psoriasis, Systemic Lupus Erythrematosis, Wegener's Granulamatosis, etc.

Within the scope of the present invention, it was demonstrated that the antibody induced response to the antigenic composition according to the invention is largely T-cell independent. A nude mouse model was used in this respect and nude mice were vaccinated and antibody responses measured to evaluate the Aβ-specific antibody response induced by the antigenic composition according to the invention in the immunized nude mice. The nude mice carry the Foxn1nu mutation and as a consequence, have reduced T-cell function due to the lack of a proper thymus.

As is shown in the following Examples, the antigenic composition according to the invention induced a robust and lasting anti-Aβ IgG response in the mouse model. The persistence of the antibody response was similar in wild type and nude mice, which is a clear indication that the mechanism of action is largely independent of T-cells. Specifically, vaccination of nude mice induced very similar antibody production kinetics when compared to wild type mice, albeit with a higher antibody titer with a similar IgG profile. The persistence of the antibody response and the IgG isotype distribution were similar in Wt and nude mice, which is another indication that these parameters are independent on T-cells in the context of ACI-24 vaccination.

The results from the nude mice study, which are indicative of a T-cell independent mechanism of action for the production of Aβ- antibodies in response to the treatment with the antigenic composition according to the invention, are further supported by examining the Aβ specific antibody response in CD4 depleted and non-depleted mice. The antigenic composition according to the invention may be compared to a vaccine known to be dependent on CD4⁺ T-cells (T helper cells), such as, for example, ovalbumin and aluminium hydroxide (OVA/Alum).. In such a comparision, it could be demonstrated that the antigenic composition according to the invention induces strong anti-Aβ antibody titers similar to those seen in mice not depleted of CD4 and thus a T-cell independent antibody response against Aβ.

From the above it follows that the antigenic composition according to the invention can induce robust anti-Aβ antibody titers without the presence of T-cells, specifically T helper cells, and that therefore classifies as a so-called TI independent antigen/vaccine.

The T-cell independent mechanism of action is favorable in terms of reduction of the risk of inappropriate T-cell activation and associated unwanted inflammatory responses such as encephalitis Further, the T-cell independent mechanism of action presents a low clinical safety risk and thus makes the antigenic composition of the invention especially suitable for a therapeutic use.

In a further aspect of the invention, the antigenic composition of the invention can be used to overcome immune tolerance in human AD patients. This aspect of the invention is supported by the following Examples, where it was demonstrated in a monkey model that the antigenic composition of the invention induces an anti-Aβ IgG response in cynomolgus monkeys. The lowest dose inducing an anti-Aβ IgG response was in the range of between 50 µg and 150 µg, particularly between 65 µg and 120 65 µg, particularly between 70 µg and 100 µg, particularly beteen 75 µg and 80 µg of modified antigenic peptide.

In a further embodiment, the invention thus provides a method for overcoming the immune tolerance in a human AD patient, particularly a human AD patient who has a T-cell deficiency, particularly a CD4 T-cell deficiency, comprising administering to said patient a therapeutically effective dose of an antigenic composition according to the present invention, particularly an antigenic composition comprising between 50 µg and 150 µg, particularly between 65 µg and 120 65 µg, particularly between 70 µg and 100 µg, particularly between 75 µg and 80 µg of modified antigenic peptide, particularly a palmitoylated antigenic peptide, particularly a palmitoylated Aβ1-15 antigenic peptide.

Sequence Listing:
- SEQ ID NO: 1: Control Sequence T5: Tau 379-408 [pS396, pS404]
- SEQ ID NO: 2: Sequence 1 (T1): Tau 5-20 [pY18 ]
- SEQ ID NO: 3: Sequence 8 (T8): Tau 206-221 [pT212, pS214]
- SEQ ID NO: 4: Sequence 9 (T9): Tau 196-211 [pS202, pT205
- SEQ ID NO: 5: Sequence 3 (T3): Tau 393-408 [pS396, pS404]
- SEQ ID NO: 6: Sequence 4 (T4): Tau 401-418 [pS404, pS409]
- SEQ ID NO: 7: Sequence 2 (T2): Tau 200-216 [pS202+ pT205 & pT212+pS214]
- SEQ ID NO: 8: antigenic peptide Aβ 1-15
- SEQ ID NO: 9: antigenic peptide Aβ 1-16
- SEQ ID NO: 10: antigenic peptide Aβ 1-16(?14)
- SEQ ID NO: 11: antigenic peptide Aβ 4-11
- SEQ ID NO: 12: antigenic peptide Aβ 22-35
- SEQ ID NO: 13: antigenic peptide Aβ 29-40
- SEQ ID NO: 14: antigenic peptide Aβ 1-5

### EXAMPLES

### EXAMPLE 1: Antibody response in female nude mice

The objective of this study was to evaluate the Amyloid-beta (Aβ)-specific antibody response induced by ACI-24 batch in female nude mice. The nude mice carry the Foxn1^{nu} mutation, have a reduced T-cell function due to the lack of properly functioning thymic gland. Thus, the aim of this study was to analyze whether the antibody response induced by ACI-24 is T-cell independent.

Nude mice with a C57BL/6 background and corresponding littermates at an age of 11 or 13 weeks were injected subcutaneously (s.c.). Mice were immunized 3 times with 2-week intervals and were bled 1 week after each immunization. Total anti-Aβ IgG responses were measured by ELISA. In addition, the isotype pattern of the anti-Aβ IgG was analyzed.

To verify the absence of T-helper cells in the nude mice, the percentage of CD3⁺ CD4⁺ cells was evaluated by fluorescence-activated cell sorter (FACS).

### 1.1 Methods

### Preparation of the vaccine ACI-24

Liposomes were prepared by solubilizing dimyristoyl phosphatidyl choline (DMPC, Lipoid, Switzerland), dimyristoyl phosphatidyl glycerol (DMPG, Lipoid, Switzerland), and cholesterol (Solvay, Netherlands) at molar ratios 9:1:7 in EtOH at 40-60°C. Palmitoylated Aβ1-15 (Pal1-15, human sequence of Aβ) was dissolved in 1% octyl-β-D-glucopyranoside (β-OG, Pentapharm) at 60 °C in PBS pH 11.5. The lipid/ethanol solution was sterile filtered and injected into the β-OG/PBS solution and immediately diluted in PBS. The generated liposomes were sterile filtered and concentrated by ultra-diafiltration to reach determined lipid concentration. Monophosphoryl Lipid A (MPLA, Avanti Polar Lipids, Inc. AL, USA), was added at the same time as the phospholipids.

### 1.1.2 Immunizations

At JSW Life Sciences, nude mice (*B6.Cg-Foxn1^{nu}*/*J*) with a C57BL/6 background and corresponding littermates (11 mice/group) received s.c. injections of ACI-24 or PBS on three occasions with a 2-week interval between each administration (day 0, 14, 28) according to Table 1. Plasma samples from the mandibular plexus were collected 7 days before and 7, 21, 35, 56, 70, 84 and 98 days after the first injections. Aβ1-42-specific IgG and IgM antibody titers and IgG isotype patterns were determined by ELISA. Blood samples were also collected on either day 77 or on day 63 after the first immunization for FACS analysis to determine the percentage of CD3⁺/CD4⁺ cells.

### Quantification of Aβ-specific antibodies

Aβ1-42-specific IgG antibodies were determined by ELISA. Plates were coated with 10 µg/ml of Aβ1-42 (Bachem, Bubendorf, ) overnight at 4°C. After washing with0.05% Tween 20 in PBS and □ Switzerland blocking with 1% BSA, serial dilutions of sera were added to the plates and incubated at 37°C for 2 hour. After washing, plates were incubated with alkaline phosphatase (AP) conjugated anti-mouse IgG antibody (Jackson Immunoresearch West Grove, PA, USA) horse radish peroxidase (HRP) conjugated anti-mouse IgM or IgG isotype specific antibodies (IgG1-AP, IgG2a-biotin, IgG3-biotin (all from Pharmingen BD, San Diego, CA, USA) or IgG2b-HRP (Zymed Laboratories, San Francisco, CA) for 2 hours at 37°C. For IgG2a and IgG3 antibodies, an additional incubation for 45 minutes at room temperature with Streptavidin-HRP was carried out. After final washing, plates were incubated with AP substrate (pNPP) or HRP substrate (ABTS) and read at 405 nm using an ELISA plate reader. Results are expressed by reference to serial dilutions of a commercially available antibody (6E10, Covance, Emeryville, CA, USA) or as optical density (O.D).

### 1.1.3 CD3⁺/CD4⁺ cell quantification

Mouse blood samples were lysed with ammonium chloride until clear, then centrifuged at 400x g for 7 minutes and pellets were resuspended in PBS containing EDTA. Then cells were blocked with CD16/CD32 blocking reagent and stained with CD4 (PE conjugate) and CD3 (PE-Cy5) antibodies for 30 min at 4°C. Samples were washed with PBS, resuspended in fixative solution (DB Cellfix diluted 1:40 in BD FACSFlow) and acquired on a BD FACS Calibur cytometer. The percentage of gated cells, which stained positive for CD3⁺ and CD4⁺ (T-helper cells) was evaluated.

The following table 1 illustrates the allocation of mice to different groups.

**Table 1. Allocation of mice to different groups**

| The following table illustrates the allocation of mice to different groups. | | | | | | | |
|---|---|---|---|---|---|---|---|
| **Group** | **No of animals and gender** | **Treatment/ Volume^{a}** | **Vaccine batch** | **Process** | **Route of Administration^{b}** | **Dose level Quantity** of **Pal1-15 µg/dose^{c}** | **Quantity of MPLA µg/dose^{d}** |
| 1 m | 11 nude female ice | PBS | 107K23 14 | NA^{e} | s.c. | 0 | 0 |
| 2 m | 11 nude female ice | ACI-24-125 0.2 ml | ACI24 0408-A | D | S.C. | 77 | Not detectable |
| 2 | 11 Wt female mice | ACI-24-125 0.2 ml | ACI-24 0408-A | D | s.c. | 77 | detectable |

| | | | | | | | |
|---|---|---|---|---|---|---|---|
| ^{a}: theoretical volume ^{b}:s.c.: subcutaneous ^{c}: measured quantity determined after analysis ^{d}: theoretical quantity ^{e}: Not applicable | | | | | | | |

1.2 *Results*

None of the animals died prematurely and no side effects due to the treatment had to be reported. For all B6.Cg-Foxn1^{nu}/J animals, the typical nude phenotype was present, while the Wt (wild type) littermates had a normal fur.

### CD3⁺/CD4⁺cell quantification

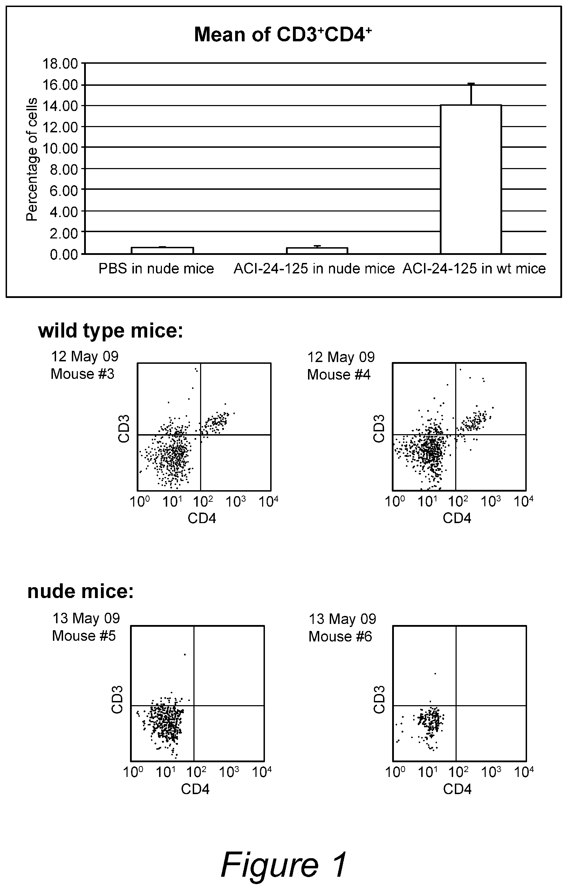

CD3⁺/CD4⁺ staining followed by FACS analysis revealed significant reduction in T-helper cell counts (CD3⁺/CD4⁺ cells) in nude mice, compared to Wt animals (Figure 1).

The percent gated cells, which were stained positive for CD3 and CD4, of nude mice receiving either PBS or ACI-24 and Wt mice receiving ACI-24. Each column represents mean and SD for groups of 11 mice. Right panel: schematic representation of FACS analysis in two mice of nude and Wt groups.

### Immune response analysis

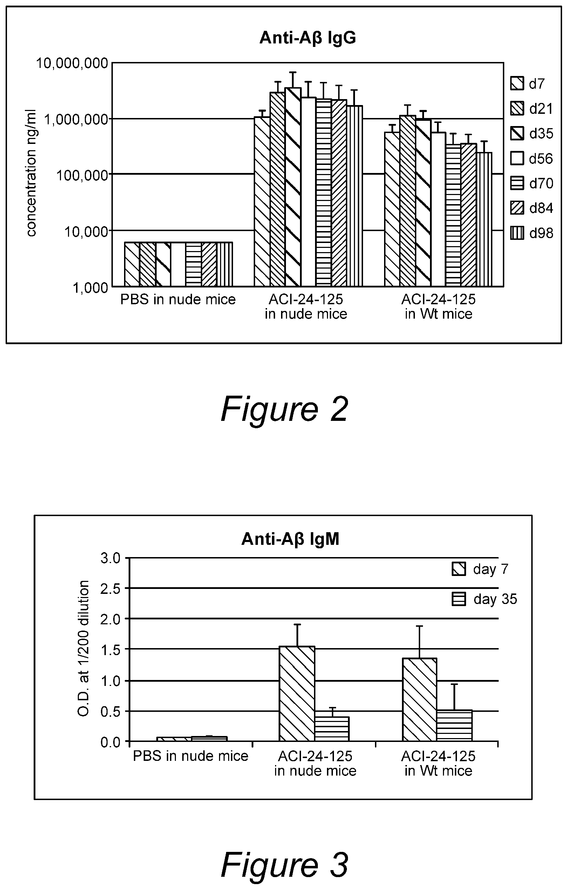

The titers of wt and nude mice were analyzed to verify whether the response induced by ACI-24 is independent on T cell function. In both Wt mice and nude mice, the ACI-24 vaccine (batch GMP-like#4) induced a robust anti-Aβ IgG responses in comparison to the PBS (Figure 2; 2-way ANOVA immunization: P<0.0001, bleeding P<0.015 and immunization*bleeding, P<0.015). Following the first immunization, the titers of the nude mice were similar to that of the Wt mice. At all the other bleeding days, the titers of the nude mice were significantly higher than of the Wt mice (2-way ANOVA). However, when analyzed by 1-way ANOVA, the nude mice titers remained significantly higher only at day 35.

To verify the boosting effect, the titers following each injection (i.e. day 7, 21 and 35) were analyzed. In nude mice treated with ACI-24, there was no significant difference between these bleeding days indicating no boosting effect. Within the group of the Wt mice treated with ACI-24, there is a boosting effect following the second injection (1-way ANOVA day 21 versus day 7).

To verify the persistence of the immune response, the titers following the last injection were analyzed (day 35, 56, 70, 84 and 98). In both the nude and Wt mice treated with ACI-24, titers remained high even 3 months after the last immunization (1-way ANOVA: no significant difference between day 7 versus day 98 for either group). In the Wt mice treated with ACI-24, the titers at day 98 decreased slight but significantly when compared to the titers at day 35 whereas no significant decrease was observed for the nude mice.

Taken together, these results show that ACI-24 induces a robust and persistent antibody response in both nude and Wt mice.

Analysis of anti-Aβ IgG antibodies in the plasma of nude and Wt mice receiving ACI-24 vaccine 7, 21, 35, 56, 70, 84 and 98 days after the first immunization. Results are expressed as mean + standard deviation obtained in groups of 11 mice.

The ACI-24 vaccine induced an anti-Aβ IgM antibody response significantly different from PBS at day 7 and 35 in both nude and Wt mice (Figure 3). In both groups, the IgM titers were higher at day 7 after the first immunization and deceased significantly at day 35. The IgM antibody responses were similar in the nude and Wt mice treated with ACI-24 at the same bleeding days.

Analysis of anti-Aβ IgM antibodies in the plasma of nude and Wt mice receiving ACI-24 vaccine 7 and 35 days after the first immunization. Results are expressed as mean + standard deviation obtained in groups of 11 mice.

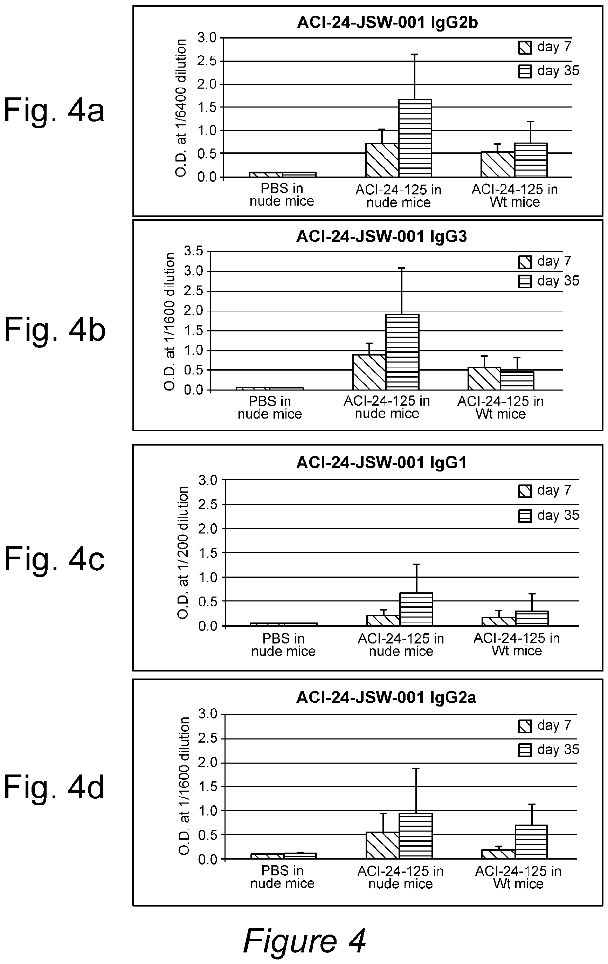

In both nude and Wt groups, ACI-24 vaccine induced anti-Aβ antibodies of IgG1, IgG2a, and IgG2b and IgG3 isotypes with IgG2b as the major subclass (Figure 4).

The profile of IgG subclasses was similar in Wt and nude mice.

Analysis of anti-Aβ IgG1, IgG2a, IgG2b, IgG3 antibodies in the plasma of nude and Wt mice receiving ACI-24 vaccine 7 and 35 days after the first immunization. Results are expressed as O.D. at a dilution of 1/200 (lgG1) 1/1600 (IgG2a) and 1/6400 (IgG2b) and 1/1600 (IgG3) showing mean + standard deviation obtained in groups of 11 mice.

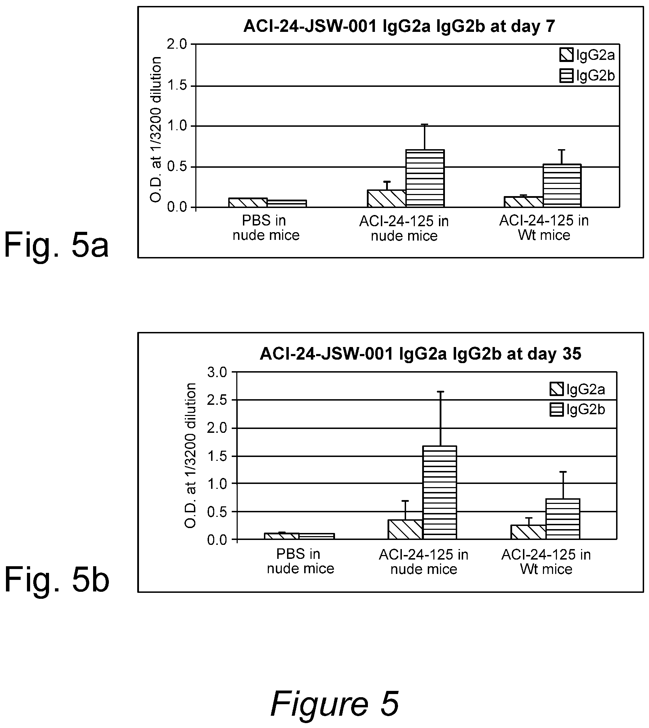

When comparing the IgG2a and IgG2b titers at the same dilution, IgG2b antibodies were dominating in both Wt and nude mice (Figure 5). In accordance with the IgG titers, the IgG2b titers in the nude mice were significantly higher than in the Wt mice at day 7 and 35.

Analysis of anti-Aβ IgG2a and IgG2b antibodies in the plasma of nude and Wt mice receiving ACI-24 vaccine 7 and 35 days after the first immunization. Results are expressed as O.D. at a dilution of 1/3200 showing mean + standard deviation obtained in groups of 11 mice.

Despite the small percentage of CD3⁺ and CD4⁺ cells in nude mice, ACI-24 vaccine induced a robust anti-Aβ IgG response. The persistence of the antibody response and the IgG isotype distribution were similar in Wt and nude mice suggesting that these parameters are independent on T-cells in the context of ACI-24 vaccination. Compared to immune-competent mice, ACI-24 immunization induced an identical antibody kinetic, a higher antibody titer with similar IgG profile in T-cell deficient mice indicating that ACI-24 induced a T-cell-independent antibody response in both nude and Wt mice.

### EXAMPLE 2: Potency of ACI-24 in CD4 depleted mice- depletion occurs once before immunization

The objective of this study was to evaluate the amyloid-beta (Aβ)-specific antibody response induced by ACI-24 in CD4 depleted and non-depleted C57BL/6 mice. This vaccine was compared to ovalbumin (OVA) and aluminium hydroxide (Alum), a vaccine known to be dependent on CD4⁺ T cells (T-helper cells). C57BL/6 mice were injected intra-peritoneal (i.p.) with anti-CD4 monoclonal antibody and subcutaneously (s.c.) immunized with ACI-24 or OVA/Alum after three days. Blood samples were collected at day 4, 7, 14 and 21 post-immunization. Fluorescence-activated cell sorter (FACS) was performed at day 21 post-immunizations to confirm CD4 depletion. Total anti-Aβ IgG responses were measured by ELISA for each of the bleedings.

### 2.1 Methods

### 2.1.1 Preparation of the ACI-24 vaccine

Liposomes were prepared as described in Example 1.1..

### 2.1.2 Preparation of the OVA/Alum vaccine

OVA vaccine was prepared by diluting 10 mg of OVA (Sigma) in 1 ml of sterile water and further diluted in PBS to obtain a concentration of 1 mg/ml. A final solution of 0.5 mg/ml OVA to 5 mg/ml Alum (Alhydrogel 2%; Brenntag Biosector, Frederikssund, Denmark) was prepared in 0.9% NaCl.

### 2.1.3 CD4 depletion

Depletion of CD4⁺ cells was achieved by i.p. injection (200µl) of anti-CD4 antibody (Clone YTS191.1; AbD Serotec, Oxford, UK) diluted in PBS (100 µg/dose/mouse), three days before immunizations (day -3). FACS analysis was performed in all animals at day 21 post-immunizations.

### 2.1..4 Immunizations

C57BL/6 adult mice (5 females/group) received s.c. injections of 200 µl/mouse of ACI-24 or OVA/Alum at day 0 (three days after CD4 depletion) according to Table 2. Bleedings were carried out at day 4, 7 and 14, as well as at sacrifice day (day 21). Plasma from all bleedings was prepared and anti-Aβ1-42-specific IgG and anti-OVA-specific IgG were determined by ELISA.

### 2.1.5 FACS analysis

Blood from all animals was collected at day 21 post-immunizations for FACS analysis to confirm CD4 depletion. Red blood cell lysis was performed by incubating blood preparations with ACK Lysing Buffer (Invitrogen, Paisley, UK) for 10 minutes at 4°C. Remaining cells were washed in cold PBS and incubated with APC-labeled anti-CD3 (clone KT3; AbD Serotec) and FITC-labeled anti-CD4 (clone RM4-5; AbD Serotec) at 4°C in the dark for 30 minutes. Cells were then washed in cold PBS, resuspended in 1% paraformaldehyde in PBS and acquired with a CyAn ADP flow cytometer analyzer (Beckman Coulter GMBH, Lausanne, Switzerland). The percentage of gated cells that stained positive for CD3⁺/CD4⁺ was processed and determined using Summit V4.3.01 software (Beckman Coulter GMBH).

Table 2 illustrates the allocation of mice to different groups.

**Table 2. Allocation of mice to different groups.**

| **Group** | **No of animals and gender** | **Treatment & Volume^{a}** | **Treatment & Volume^{b}** | **Process** | **Dose level quantity of Pal1-15 (µg/dose^{c})** | **Quantity ^{of} MPLA (µg/dose ^{c, d})** | **Quantity of OVA/Alum (µg/dose)** |
|---|---|---|---|---|---|---|---|
| F | female s | 5 CD4 depletio n (200µl) | ACI-24 (200µl) | **D** | 83.2 | 15 | - |
| G | 5 female s | - | (200µl) | **D** | ACI-24 83.2 | 15 | - |
| H | female s (200µl) | CD4 depletio n | OVA/Alum (200µl) | - | - | - | 100/100 0 |
| I | 5 female s | - | OVA/Alum (200µl) | - | - | - | 100/100 0 |

| | | | | | | | |
|---|---|---|---|---|---|---|---|
| ^{a}: i.p.: intraperitoneal ^{b}:s.c.: subcutaneous ^{c}: measured quantity determined after analysis ^{d}: sum of MPLA congeners A + B | | | | | | | |

### 2.1.6 Quantification of Aβ-specific antibodies

Aβ1-42-specific IgG response was determined by ELISA. Plates were coated with 10 µg/ml of Aβ1-42 (Bachem, Bubendorf, ) overnight at 4°C. After washing with PBS-0□Switzerland.05% Tween 20 and blocking with 1% BSA, serial dilutions of sera were added to the plates and incubated at 37°C for 2 hours. After washing, plates were incubated with alkaline phosphatase (AP) conjugated anti-mouse IgG antibody (Jackson Immunoresearch West Grove, PA, USA) for 2 hours at 37°C. After final washing, plates were incubated with AP substrate (pNPP) and read at 405 nm using an ELISA plate reader. Results are expressed by reference to serial dilutions of a commercially available antibody (6E10, Covance, Emeryville, CA, USA).

### 2.1.7 Quantification of OVA-specific antibodies

OVA-specific IgG antibodies were determined by ELISA. overnight at 4°C. After□Plates were coated with 30 µg/ml of OVA (Sigma, A5503) washing with PBS-0.05% Tween 20 and blocking with 1% BSA, serial dilutions of sera were added to the plates and incubated at 37°C for 2 hours. After washing, plates were incubated with AP conjugated anti-mouse IgG total antibody (Jackson Immunoresearch, PA, USA) for 2 hours at 37°C. After final washing, plates were incubated with pNPP and read at 405 nm using an ELISA plate reader. Results were expressed as optical density (O.D.).

### 2.2 Results

None of the animals died prematurely and no side effects due to the CD4 depletion, ACI-24 or OVA/Alum immunizations were observed. Body weights between the groups were similar, although significant changes were observed at day 21 between depleted and non-depleted ACI-24 immunized mice (2-way ANOVA, P<0.05). Other significant changes were observed between groups, although these were not between comparable treatments and therefore deemed not relevant.

Analysis of the body weight of CD4 depleted and non-depleted ACI-24 and OVA/Alum immunized mice. Results are expressed as mean + standard deviation with n=5 for each of the groups.

### 2.2.1 CD3⁺/CD4⁺cell quantification

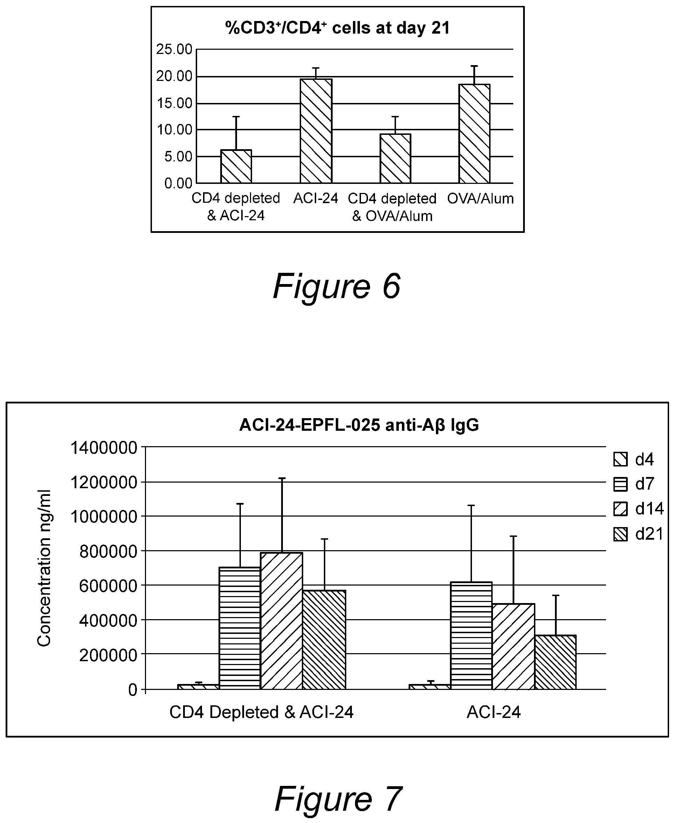

CD3⁺/CD4⁺ staining followed by FACS analysis revealed lower CD3⁺/CD4⁺ (T-helper cells) counts in animals of groups which had CD4 depleted (Figure 6). A significant difference was found in between CD4 depleted and non-depleted OVA/Alum immunized mice (Figure 6, 1-way ANOVA P<0.05).

### 2.2.2 Immune responses following A CI-24 immunization

The titers of anti-Aβ in CD4 depleted and non-depleted mice were analyzed to verify whether the response induced by ACI-24 is independent on CD3⁺/CD4⁺ (T helper cells). The ACI-24 vaccine induced a robust anti-Aβ IgG response starting from day 7 up until day 21. Overall, CD4 depleted mice recorded higher, albeit non-significant, anti-Aβ titers from day 7 until the last bleed (day 21) (Figure 7).

Titers of anti-Aβ IgG antibodies in the plasma of C57BL/6 mice at days 4, 7, 14 and 21 after ACI-24 immunization. Results are expressed as mean + standard deviation obtained in groups of 5 mice.

### 2,2.3 Immune responses following OVA/Alum immunization

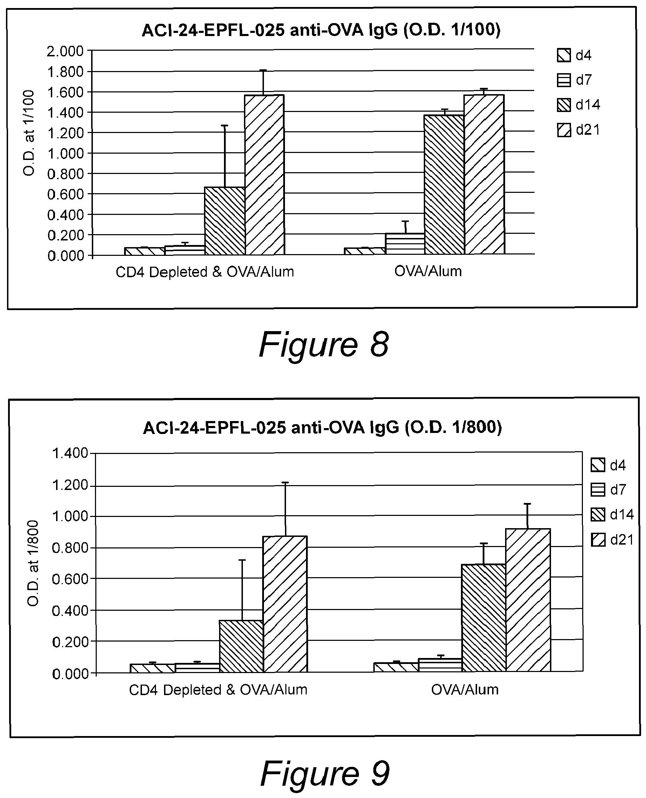

Anti-OVA IgG titers were analyzed to verify whether CD4 depletion had been achieved, as high IgG titers against OVA are dependent on CD3⁺/CD4⁺ cells (T helper cells). Negligible titers were observed at day 4 and day 7 in both CD4 depleted and non-depleted animals when O.D. was recorded at 1/100 or 1/800. At day 14 the anti-OVA IgG titers were significantly lower in CD4 depleted mice when compared to non-depleted mice (Figure 8, 2-way ANOVA P<0.001); a finding confirmed when the O.D. was recorded at 1/800 (Figure 9, 2-way ANOVA P<0.05). Anti-OVA IgG titers at day 21 were identical in CD4 depleted and non-depleted mice, suggesting that the CD4 T cell population recovered during this time frame (from day -3 to day 21).

Titers of anti-OVA IgG antibodies in the plasma of C57BL/6 mice at days 4, 7, 14 and 21 after OVA/Alum immunization. Results are expressed as OD of the mean + standard deviation obtained in groups of 5 mice when read at 1/100.

Titers of anti-OVA IgG in the plasma of C57BL/6 mice at days 4, 7, 14 and 21 after OVA/Alum immunization. Results are expressed as OD of the mean + standard deviation obtained in groups of 5 mice when read at 1/800.

Despite the small percentage of CD3⁺/CD4⁺ cells in CD4 depleted mice, ACI-24 vaccine induced strong anti-Aβ titers similar to non-depleted mice. As expected, IgG titers against OVA in CD4 depleted mice were significantly lower than those observed in non-depleted mice 14 days after immunization, confirming that the CD4 depletion had a direct effect on a classical T-dependent antibody response. Altogether, results show that the CD4 depletion was successful, and that in C57BL/6 mice ACI-24 induces a T-cell independent antibody response against Aβ.

### EXAMPLE 3: Potency of ACI-24 in CD4 depleted mice - depletion occurs each week before immunization

The objective of this study was to evaluate the amyloid-beta (Aβ)-specific antibody response induced by ACI-24 in CD4 depleted and non-depleted C57BL/6 mice. This vaccine was compared to ovalbumin (OVA) and aluminium hydroxide (Alum), a vaccine known to be dependent on CD4⁺ T cells (T-helper cells). C57BL/6 mice were injected weekly with anti-CD4 monoclonal antibody via intra-peritoneal (i.p.) route and subcutaneously (s.c.) immunized with ACI-24 or OVA/Alum three days after the first anti-CD4 injection. Blood samples were collected at day 7, 14 and 21 post-immunization. Fluorescence-activated cell sorter (FACS) was performed at each of the bleeding days to confirm CD4 depletion. Total anti-Aβ IgG and anti-OVA responses were measured by ELISA for each of the bleedings.

### 3.1 Methods

### 3.1.1 Preparation of the ACI-24 vaccine

Liposomes were prepared as described in Example 1.1

### 3.1.2 Preparation of the OVA/Alum vaccine

OVA vaccine was prepared by diluting 10 mg of OVA (Sigma) in 1 ml of sterile water and further diluted in PBS to obtain a concentration of 1 mg/ml. A final solution of 0.5 mg/ml OVA to 5 mg/ml Alum (Alhydrogel 2%; Brenntag Biosector, Frederikssund, Denmark) was prepared in 0.9% NaCl.

### 3.1.3 CD4 depletion

Depletion of CD4⁺ cells in group A and C was achieved by i.p. injection (200µl) of anti-CD4 antibody (Clone YTS191.1; AbD Serotec, Oxford, UK) diluted in PBS (100 µg/dose/mouse), three days before immunizations (day -3) and weekly thereafter (day 4, 11 and 18). Group B and D were injected with PBS. FACS analysis was performed in all animals at all bleeding days (day 7, 14 and 21).

### 3.1.4 Immunizations

C57BL/6 adult mice (8 females/group) received s.c. injections of 200 µl/mouse of ACI-24 or OVA/Alum three days after the first CD4 depletion (day 0) according to Table 3. Bleedings were carried out at day 7, 14 and day 21. Plasma from all bleedings was prepared and anti-Aβ1-42-specific IgG and anti-OVA-specific IgG were determined by ELISA.

Blood from all animals was collected at day 7, 14 and 21 post-immunization for FACS analysis to confirm CD4 depletion. Red blood cell lysis was performed by incubating blood preparations with ACK Lysing Buffer (Invitrogen, Paisley, UK) for 10 minutes at 4°C. Remaining cells were washed in cold PBS and incubated with APC-labeled anti-CD3 (clone KT3; AbD Serotec) and FITC-labeled anti-CD4 (clone RM4-5; AbD Serotec) at 4°C in the dark for 30 minutes. Cells were then washed in cold PBS, resuspended in 1% paraformaldehyde in PBS and acquired with a CyAn ADP flow cytometer analyzer (Beckman Coulter GMBH, Lausanne, Switzerland). The percentage of gated cells that stained positive for CD3⁺/CD4⁺ was processed and determined using Summit V4.3.01 software (Beckman Coulter GMBH).

Table 3 illustrates the allocation of mice to different groups.

**Table 3. Allocation of mice to different groups.**

| **Group** | **No of animals and gender** | **Treatment & Volume^{a}** | **Treatment & Volume^{b}** | **Process** | **Dose level quantity of Pal1-15 (µg/dose^{c})** | **Quantity of MPLA (µg/dose^{c, d})** | **Quantity of OVA/Alum (µg/dose)** |
|---|---|---|---|---|---|---|---|
| A | female s (200µl) | CD4 depletio n | ACI-24 (200µl) | **D** | 83.2 | 15 | - |
| B | 8 female s | PBS (200µl) | (200µl) | **D** | ACI-24 83.2 | 15 | - |
| C | female s (200µl) | CD4 depletio n | OVA/Alum (200µl) | - | - | - | 100/100 0 |
| D | 8 female s | PBS (200µl) | (200µl) | - | OVA/Alum - | - | 100/100 0 |

| | | | | | | | |
|---|---|---|---|---|---|---|---|
| ^{a}: i.p.: intraperitoneal ^{b}:s.c.: subcutaneous ^{c}: measured quantity determined after analysis ^{d}: sum of MPLA congeners A + B | | | | | | | |

### 3.1.5 Quantification of Aβ-specific antibodies

Aβ1-42-specific IgG response was determined by ELISA. Plates were coated with 10 µg/ml of Aβ1-42 (Bachem, Bubendorf, ) overnight at 4°C. After washing with PBS-0.05% Tween 20 and□Switzerland blocking with 1% BSA, serial dilutions of sera were added to the plates and incubated at 37°C for 2 hours. After washing, plates were incubated with alkaline phosphatase (AP) conjugated anti-mouse IgG antibody (Jackson Immunoresearch West Grove, PA, USA) for 2 hours at 37°C. After final washing, plates were incubated with AP substrate (pNPP) and read at 405 nm using an ELISA plate reader. Results are expressed by reference to serial dilutions of a commercial available antibody (6E10, Covance, Emeryville, CA, USA).

### 3.1.6 Quantification of OVA-specific antibodies

OVA-specific IgG antibodies were determined by ELISA. Plates were coated with 30 µg/ml of OVA (Sigma, A5503) overnight at 4°C. After washing with PBS-0.05% Tween 20 and blocking with 1% BSA, serial dilutions of sera were added to the plates and incubated at 37°C for 2 hours. After washing, plates were incubated with AP conjugated anti-mouse IgG total antibody (Jackson Immunoresearch, PA, USA) for 2 hours at 37°C. After final washing, plates were incubated with pNPP and read at 405 nm using an ELISA plate reader. Results were expressed as optical density (O.D.).

### 3.2 Results

Although one animal was sacrificed due to reasons unrelated to the treatment/immunization (#3 in Group C), no animals died prematurely and no side effects due to the CD4 depletion, ACI-24 or OVA/Alum immunizations were observed. Body weights between the groups were also unaffected by the different treatments.

### 3.2.1 CD3⁺/CD4⁺cell quantification

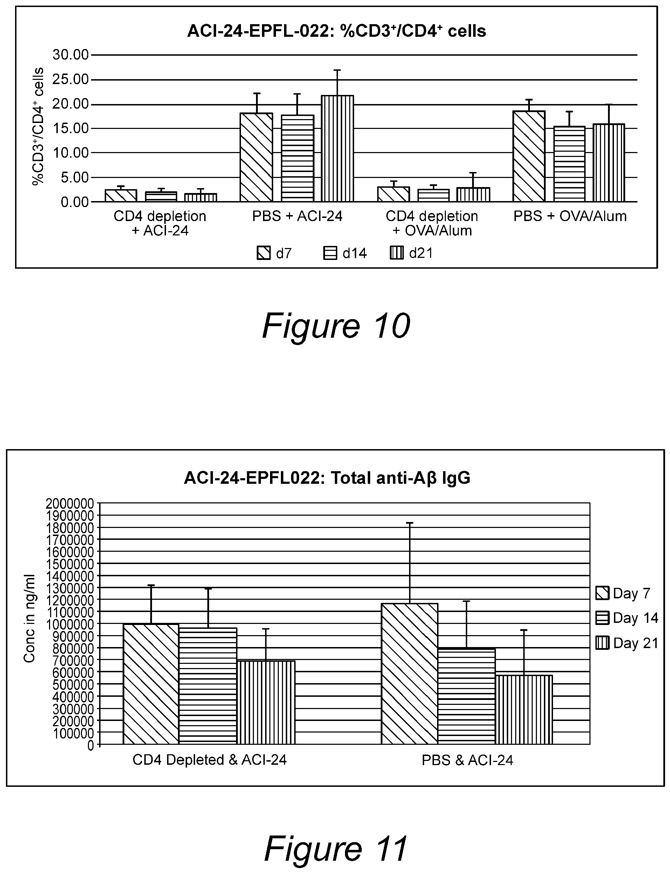

CD3⁺/CD4⁺ staining followed by FACS analysis revealed significantly lower CD3⁺/CD4⁺ (T-helper cells) counts in animals of CD4 depleted groups for all bleeding days (P<0.001, 2 way ANOVA) (Figure 10).

The percentage of gated cells that stained positive for CD3 and CD4 in ACI-24 and OVA/Alum immunized groups at all day 7, 14 and 21. Results are expressed as % of CD3⁺/CD4⁺ mean + standard deviation obtained in groups of 10 mice.

### 3.2.2 Immune responses following A CI-24 immunization

The titers of anti-Aβ in CD4 depleted and non-depleted mice were analyzed to verify whether the response induced by ACI-24 is independent on CD3⁺/CD4⁺ (T helper cells). The ACI-24 vaccine induced a robust anti-Aβ IgG response starting from day 7 up until day 21. No significant difference in the anti-Aβ titers was observed when comparing CD4 depleted mice with non-depleted mice (Figure 11).

Titers of anti-Aβ IgG antibodies in the plasma of C57BL/6 mice at days 7, 14 and 21 after ACI-24 immunization. Results are expressed as mean + standard deviation obtained in groups of 10 mice.

### 3,2.3 Immune responses following OVA/Alum immunization

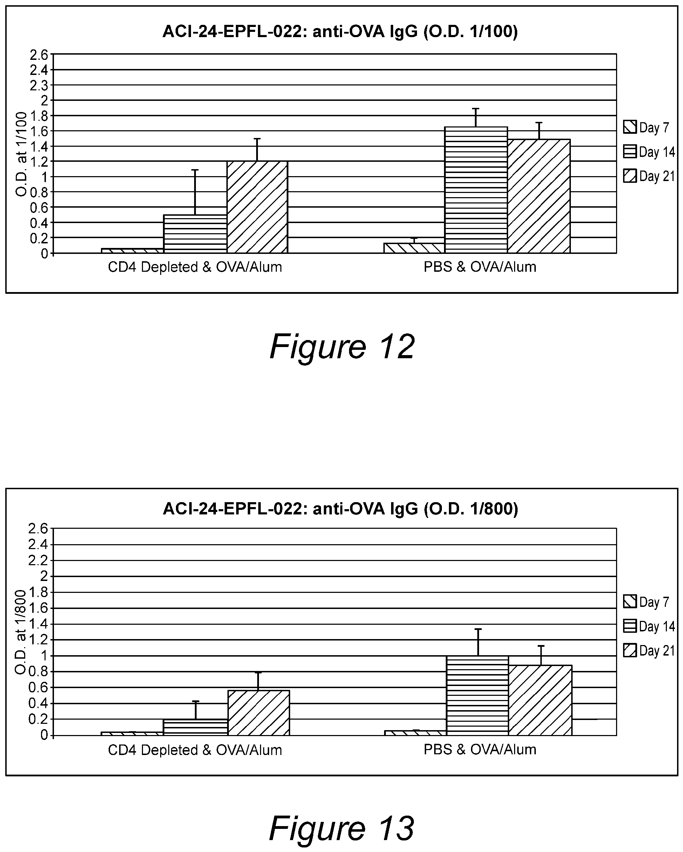

Anti-OVA IgG titers were analyzed to confirm CD4 depletion, as high IgG titers against OVA are dependent on CD3⁺/CD4⁺ cells (T helper cells). Negligible titers were observed at day 7 in both CD4 depleted and non-depleted animals when O.D. was recorded at 1/100 or 1/800. At day 14, the anti-OVA IgG titers were significantly lower in CD4 depleted mice when compared to non-depleted mice (Figure 12, 2-way ANOVA P<0.001); a finding confirmed at day 14 and 21 when the O.D. was recorded at 1/800 (Figure 13, 2-way ANOVA P<0.001 and P<0.01 respectively).

Titers of anti-OVA IgG antibodies in the plasma of C57BL/6 mice at days 7, 14 and 21 after OVA/Alum immunization. Results are expressed as OD of the mean + standard deviation obtained in groups of 10 mice when read at 1/100. iters of anti-OVA IgG in the plasma of C57BL/6 mice at days 7, 14 and 21 after OVA/Alum immunization. Results are expressed as OD of the mean + standard deviation obtained in groups of 10 mice when read at 1/800. Despite the small percentage of CD3⁺/CD4⁺ cells in CD4 depleted mice, ACI-24 vaccine induced strong anti-Aβ titers similar to non-depleted mice. As expected, IgG titers against OVA in CD4 depleted mice were significantly lower than those observed in non-depleted mice 14 (1/100 O.D.) and 21 days after immunization (1/100 and 1/800 O.D.), confirming that the CD4 depletion had a direct effect on a classical T-dependent antibody response. Altogether, results show that the CD4 depletion was successful, and that in C57BL/6 mice ACI-24 induces a CD4⁺ T-cell independent antibody response against Aβ.

### EXAMPLE 4: Immunogenicity of ACI-24 in Cynomolgus Monkeys

The objective of this study was to evaluate the immunogenicity of ACI-24 in cynomolgus monkeys *(Macaca fascicularis).* Cynomolgus monkeys were chosen for this study as the amino acid sequence of the amyloid peptide is completely homologous when comparing monkeys and humans. The monkeys express the β-amyloid (Aβ) as a self-protein and may therefore have a certain level of immunological tolerance against this peptide. We set out to analyze whether immunization with ACI-24, containing a palmitoylated peptide of human Aβ1-15 (PaI1-15), could break the immune tolerance in these monkeys.

To determine the immunogenicity of ACI-24, monkeys were given subcutaneously (s.c.) 6 doses of ACI-24 at 4-week intervals. The antibody response was analyzed by measuring anti-Aβ IgM and IgG antibody titers in the sera of immunized monkeys at different time points after vaccination by ELISA.

### 4.1 Methods

### 4.1.1 Preparation of the ACI-24 vaccine

Liposomes were prepared by solubilizing dimyristoyl phosphatidyl choline (DMPC, Lipoid, Switzerland), dimyristoyl phosphatidyl glycerol (DMPG, Lipoid, Switzerland), and cholesterol (Solvay, Netherlands) at molar ratios 9:1:7 in EtOH at 40-60°C. Palmitoylated Aβ1-15 (Pal1-15, human sequence of Aβ1-15) was dissolved in 2% octyl-β-D-glucopyranoside (β-OG, Fluka) in PBS. The lipid/ethanol solution was injected into the β-OG/PBS solution and immediately diluted in PBS. The generated liposomes were concentrated by ultra-diafiltration to reach determined lipid concentration. Monophosphoryl Lipid A (MPLA, Avanti Polar Lipids, Inc. AL, USA), was added at the same time as the phospholipids. The different vaccine preparations ACI-24-125 (R/ACI/PAL1-15/Pool/140607+180607), ACI-24-30 (Diluted/R/ACI/PAL1-15/Pool/140607+180607) and ACI-Empty (R/ACI/PaI1-15/110607/E) were prepared by Polymun Scientific GmbH (Austria) and shipped to Maccine Ltd. (Singapore). The theoretical and analytical quantities of PaI1-15, MPLA, cholesterol and DMPC are presented in the Appendix (Table 2).

### 4.1.2 Immunizations

18 naive monkeys were attributed to 1 of 5 different groups according to Table 4. Group 1 received PBS. Group 2 received empty liposomes, i.e. liposomes with MPLA but without Pal1-15 (labelled "ACI-24-empty"). 4 groups received ACI-24 containing different doses of PaI1-15, i.e. ACI-24-30, ACI-24-125, ACI-24-500 and ACI-24-1000. Monkeys were immunized subcutaneously (s.c.) 6 times every 4 weeks i.e. on days 0, 28, 56, 84, 112 and 140.

Table 4 illustrates the allocation of monkeys to different groups.

**Table 4. Allocation of monkeys to different groups**

| **Group** | **No of animals and Gender** | **Treatment^{a}/ Volume^{b}** | **Vaccine batch** | **Dose level Quantity of Pal1-15 µg/dose^{c}** | **Quantity of MPLA µg/dose^{c}** |
|---|---|---|---|---|---|
| **1** | 2 males, 1 female | PBS / 0.8 ml | Not applicable | 0 | 0 |
| **2** | 1 male, 2 females | ACI-24-empty / 0.8 ml | R/ACI/PaI1-15/110607/E | 0 | 82 |
| **3** | 3 males | ACI-24-30 /0.2 ml | Diluted/R/ACI/PAL1-15/Pool/140607+180607 | 28 | 9 |
| **4** | 2 males, 1 female | ACI-24-125 / 0.2 ml | R/ACI/PAL1-15/Pool/140607+180607 | 78 | 22 |
| **5** | 3 females | ACI-24-500/0.8 | R/ACI/PAL1-15/Pool/140607+180607 | 311 | 89 |
| | | ml | | | |
| **6** | 1 male, 2 females | ACI-24-1000 / 1.6 ml | R/ACI/PAL1-15/Pool/140607+180607 | 621 | 176 |

| | | | | | |
|---|---|---|---|---|---|
| ^{a}: theoretical dose is expressed in µg and indicated in vaccine name beyond ACI-24 ^{b}:theoretical volume ^{c}: measured quantity determined after analysis | | | | | |

### 4.1.3 Blood Sampling

Blood samples (a total of 8 ml) were collected and blood sera and white blood cells were isolated on a weekly basis starting from weeks -1 to 24.

Whole blood samples of approximately 4 ml were taken from the femoral vein into plain tubes and allowed to clot at room temperature for up to one hour. Serum was then separated by centrifugation at 4000 rpm for 10 minutes. Serum was separated into 1 ml aliquots with half the samples stored at -80 °C at Maccine. The remaining aliquots were shipped frozen to AC Immune on weeks 6, 10, 14, 18, 20 and 24 after treatment start.

Whole blood samples of approximately 4 ml were taken from the femoral vein into EDTA tubes (2 × 2 ml). Immediately after taking the blood samples, the lysis of the blood cells was controlled by Neubauer chamber and Trypan Blue extrusion. No more than 20% lysated blood cells were noted to be present on immediate assessment of the collected samples from August 2007 until the end of the study period. The tubes were placed on a blood roller following collection. One tube with 2 ml of whole blood was divided into 2 × 1 ml aliquots and frozen at -80 °C until shipped to AC Immune. White blood cells were isolated from the other 2 ml using the Histopaque gradient method. Two ml of blood was layered onto 2 ml of Histopaque-1077 (Sigma) in a 15 ml centrifuge tube. The gradient was then spun for 30 minutes at 400 g with the brake disabled. The white blood cells, found at the Histopaque /plasma interface, were harvested, washed with isotonic phosphate buffered saline and centrifuged at 400 g for 5 minutes. After two washes, the centrifuged cells were resuspended in 2 ml of cold freezing medium (70% RPMI; 20% FBS; 10% DMSO). The cells were then placed in a pre-cooled Nalgene cell freezing container and kept at -80 °C for approximately 24 hours after which both vials were placed in liquid nitrogen until shipment to AC Immune on dry ice for analysis.

### 4.1.4 Quantification of Aβ-specific antibodies

Aβ1-42-specific IgG and IgM antibodies were determined by ELISA. Plates were coated with 10 ) or 10 µg/ml of□1-42 (Bachem, Bubendorf, Switzerland□µg/ml of human Aβ Aβ1-15 (NeoMPS, France) overnight at 4°C. After washing with PBS-0.05% Tween 20 and blocking with 1% BSA, serial dilutions of sera were added to the plates and incubated at 37°C for 2 hours. After washing, plates were incubated with horse radish peroxidase (HRP) conjugated anti-monkey IgG or IgM antibodies (KPL Inc, Gaithersburg, Maryland, USA) for 2 h at 37°C. After final washing, plates were incubated with HRP substrate (ABTS) and read at 405 nm using an ELISA plate reader.

### 4.1.5 Quantification of MPLA-specific Antibodies

MPLA-specific IgG antibodies were determined by ELISA. Plates were coated with 10 µg/ml of MPLA (Avanti Polar Lipids, Inc. AL, USA) overnight at 4°C. After washing with PBS-0.05% Tween 20 and blocking with 1% BSA, serial dilutions of sera were added to the plates and incubated at 37°C for 2 hours. After washing, plates were incubated with horse radish peroxidase (HRP) conjugated anti-monkey IgG antibodies (KPL Inc, Gaithersburg, Maryland, USA) for 2 h at 37°C. After final washing, plates were incubated with HRP substrate (ABTS) and read at 405 nm using an ELISA plate reader.

### 4.1.6 Definition of Vaccine Responders

Sera from monkeys that showed an O.D. twice as high as the O.D. obtained in the same monkey before the first immunization were considered as vaccine responders.

### 4.2 Results

### Evaluation of the Antibody Response induced by ACI-24 in Cynomolgus Monkeys

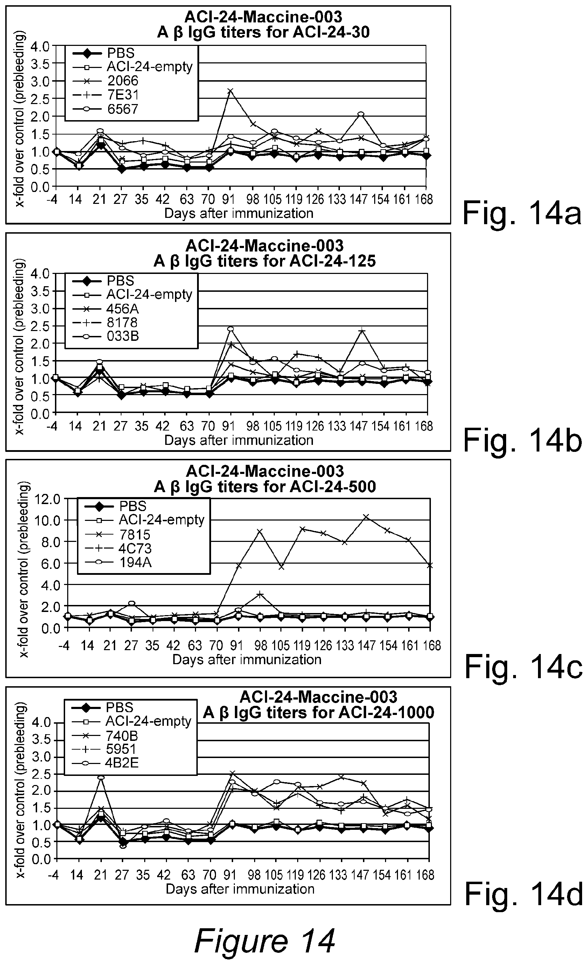

The ACI-24 vaccine injected s.c. induced robust Aβ-specific IgG in 9 monkeys (Figure 14). The responding monkeys had been vaccinated with ACI-24-30 (1 monkey, no 2066), ACI-24-125 (2 monkeys no 6178 and 033B), ACI-24-500 (all 3 monkeys, 7815, 4C73, 194A) or ACI-24-1000 (all 3 monkeys. 740B, 5951 and 4B2E). Monkeys vaccinated with PBS or empty liposomes did not show any detectable anti-Aβ IgG antibodies, as expected (Figure 14).

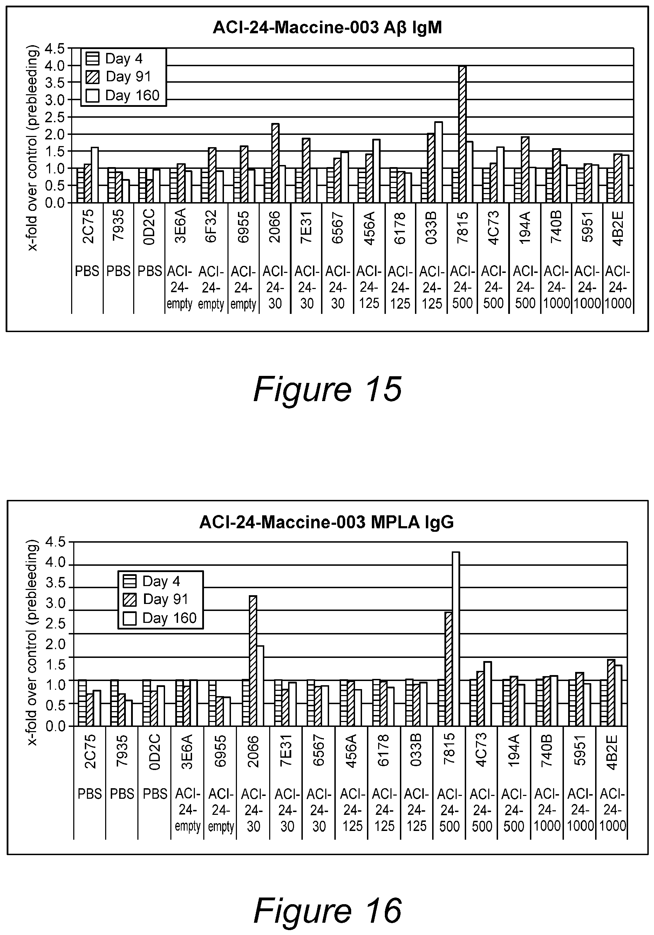

ACI-24 induced anti-Aβ IgM titers in 3 monkeys following vaccination with ACI-24-30 (1 monkey, no 2066), ACI-24-125 (1 monkey, no 033B) or ACI-24-500 (1 monkey, no 7815; Figure 15).

The ACI-24 also induced an anti-MPLA IgG response in 2 monkeys following ACI-24-30 (1 monkey, no 2066) or ACI-24-500 (1 monkey, no 7815) vaccination (Figure 16).

Analysis of anti-Aβ IgG antibodies in the sera of cynomolgus monkeys at different days after the first immunization. Monkeys were immunized with PBS, empty liposomes or ACI-24 at different doses s.c. on day 1, 28, 56, 84, 112 and 140. Results are expressed as x-fold over control (O.D. for each day, divided by the O.D. obtained before immunization). Each graph represents 1 dose group. Numbers refer to the animal number.

Analysis of anti-Aβ IgM antibodies in the sera of cynomolgus monkeys at different days after the first immunization. Monkeys were immunized with PBS, empty liposomes or ACI-24 at different doses s.c. on day 1, 28, 56, 84, 112 and 140. Results are expressed as x-fold over control (O.D. for each day, divided by the O.D obtained before immunization). Numbers refer to the animal number.

Analysis of anti-MPLA IgG antibodies in the sera of cynomolgus monkeys at different days after the first immunization. Monkeys were immunized with PBS, empty liposomes or ACI-24 at different doses s.c. on day 1, 28, 56, 84, 112 and 140. Results are expressed as x-fold over control (O.D. for each day, divided by the O.D obtained before immunization). Numbers refer to the animal number.

Thus, ACI-24 induces an anti-Aβ IgG response in cynomolgus monkeys. The ACI-24-125 vaccine containing 78 µg of Pal 1-15 was the lowest dose inducing an anti-Aβ IgG response. Thus, ACI-24 is capable of a in monkeys, suggesting that ACI-24 would also overcome immune tolerance in human AD patients.

### EXAMPLE 5: Evaluation of the antibody response induced by ACI-24 in nude mice and mice deficient for CD40L, CD14 or TLR4

The objective of this study was to evaluate the Amyloid-beta (Aβ)-specific antibody response induced by ACI-24 in nude mice and in mice deficient for CD40L, CD14 and TLR4.

Nude mice carry the Foxn1^{nu} mutation, have a reduced T cell function due to the lack of properly functioning thymic gland. Thus, one aim of this study was to analyze whether the antibody response induced by ACI-24 is T-cell independent.

CD40L knock-out mice show deficient primary and secondary antibody responses to T-dependent antigens but respond normally to T-independent antigens (Renshaw, J Exp Med, 1994, 180, 1889; Borrow, J.Exp.Med. ,1996 ,183, 2129; Xu, Immunity, 1994, 1,423). CD40L expression on CD4 T-cells is also critical for isotype switching by B cells. CD40L deficient mice were analyzed to determine whether CD40L/CD40 is required for the antibody response induced by ACI-24.

CD14 and TLR4 are molecules involved in the response to Lipopolysaccharide (LPS). TLR4 together with CD14 and the myeloid differentiation protein-2 forms a pattern recognition receptor initiating the innate immune response to LPS. Mono phosphoryl Lipid A (MPLA) is a low toxicity derivative of LPS and is used as adjuvant in the ACI-24 vaccine. The requirement for CD14 and TLR4 recognition of MPLA for the antibody response induced by ACI-24 was determined in mice deficient for either CD14 or TLR4.

All mice were injected with ACI-24 subcutaneously (s.c.). Mice were immunized 3 times with 2-week intervals and were bled at different time intervals. Total anti-Aβ IgG responses were measured by ELISA.

### 5.1 Methods

### 5.1.1 Mice

Mice deficient for TLR4 (TLR4 knock out (KO)), CD14 (CD14 KO) or CD40L (CD40L KO) and nude mice (all on a C57BI/6 background) were purchased from Jackson Laboratories (Bar Harbor, ME,USA).

### 5.1.1.1 TLR4 knock out - B6.B10ScN-Tlr4lps-del/JthJ (Jackson Stock Number: 007227)

This spontaneous mutation is a 7 kb deletion in the Tlr4 gene, which results in absence of both mRNA and protein and thus exhibits a defective response to LPS stimulation. The functionally similar Tlr4Lps-d mutation found in C3H/HeJ mice is a point mutation that causes an amino acid substitution. The allele for normal LPS response, Tlr4lps-n, occurs in most other C3H and C57BL/10 substrains and most mouse strains. This strain may be used to study the Toll signaling pathway and susceptibility to Gram-negative bacterial infection.

### 5.1.1.2 CD14 knock out - B6.129S-Cd14tm1Frm/J (Jackson Stock Number: 003726)

Mice that are homozygous null for CD14 are viable and fertile. No CD14 protein product is detected in thioglycollate elicited peritoneal (T-EP) macrophages by Western blot analysis. Unlike wildtype T-EP macrophages, T-EP macrophages derived from these animals fail to secrete TNF-alpha and IL-6 in response to lipopolysaccharide (LPS). Such a response is observed when CD14 -null T-EP macrophages are exposed to whole bacteria (in the form of E. coli bioparticles), apparently by a CD14-independent mechanism.

### 5.1.1.3 NUDE mice - B6.Cg-Foxn1nu/J (Jackson Stock Number: 000819)

The two main defects of mice homozygous for the nude spontaneous mutation (Foxn1nu, formerly Hfh11nu) are abnormal hair growth and defective development of the thymic epithelium. Although the mice appear hairless, they are born with functional but faulty hair growth follicles. Hair growth cycles and patterns are evident especially in pigmented mice but the faulty follicles do not allow the hair to properly erupt. Homozygous pups can be identified as young as 24 hours by their lack of whiskers or poorly developed, crinkled whiskers. Nude mice are also athymic caused by a developmental failure of the "thymic anlage". Consequently, homozygous nude mice lack T-cells and suffer from a lack of cell-mediated immunity. However there is not a defect in T-cell precursors, and under the right conditions some functional mature T-cells can be found especially in adult mice. Because of a defect in helper T-cell activity, responses to thymus-dependent antigens when detectable are primarily limited to IgM. Homozygous nude mice show partial defect in B cell development probably due to absence of functional T-cells.

### 5.1.1.4 CD40L knock out mice - B6.129S2-Cd40lgtm1lmx/J (Jackson Stock Number: 002770)

Mice homozygous for the targeted mutation are viable and fertile. Homozygous mutant mice show no overt phenotypic abnormalities. Percentages of B- and T-cell subpopulations are normal. Homozygotes do show selective deficiencies in humoral immunity (low basal serum isotype levels and undetectable IgE) as well as abnormal secondary antigen-specific responses to immunization with a thymus-dependent antigen. The phenotype of the mice resembles human X-linked hyper IgM syndrome.

### 5.1.2 Preparation of the vaccine ACI-24

Liposomes were prepared as described in Example 1.1

The vaccine preparation, ACI-24 (batch: ACI24-0709-A) was prepared by Polymun Scientific GmbH (Austria) and shipped to university Zurich (Switzerland)

### 5.1.3 Immunizations

C57BL/6 mice, nude mice, CD40L KO mice, CD14 KO mice and TLR4 KO mice (6 mice/group) received s.c. injections of ACI-24 on three occasions with a 2-week interval between each administration (day 0, 14, 28) according to Table 5. One group of C57BL/6 mice received PBS as negative control. Another control group received Aβ1-15 peptide. Plasma samples were collected on d0, 7, 14, 28 and 56 days after the first injection. Aβ1-42-specific IgG antibody titers were determined by ELISA.

### 5.1.4 Quantification of Aβ-specific antibodies

Aβ1-42-specific IgG antibodies were determined by ELISA. Plates were coated with 10 µg/ml of Aβ1-42 (Bachem, Bubendorf, ) overnight at 4°C. After washing with 0.05% Tween 20 in PBS and□Switzerland blocking with 1% BSA, serial dilutions of sera were added to the plates and incubated at 37°C for 2 hours. After washing, plates were incubated with alkaline phosphatase (AP) conjugated anti-mouse IgG antibody (Jackson Immunoresearch West Grove, PA, USA). After final washing, plates were incubated with AP substrate (pNPP) or HRP substrate (ABTS) and read at 405 nm using an ELISA plate reader. Results are expressed by reference to serial dilutions of a commercial available antibody (6E10, Covance, Emeryville, CA, USA).

**Table 5. Allocation of mice to different groups.**

| **Group** | **Mouse strain** | **No of animals and gender** | **Treatment/ Volume^{a}** | **Vaccine batch** | **Route of Administration^{b}** | **Dose level Quantity of Pal1-15 µg/dose ^{c}** | **Quantity of MPLA µg/dose^{c}** |
|---|---|---|---|---|---|---|---|
| 1 | C57BL/6 wildtype | 6 female mice | ACI-24 0.2 ml | ACI-24-0709-A | s.c. | 77.6 | 11.6 |
| 2 | CD40L KO | 6 female mice | ACI-ACI-24 0.2 ml | 24-0709-A | s.c. | 77.6 | 11.6 |
| 3 | Nude | 6 female mice | ACI-ACI-24 0.2 ml | 24-0709-A | s.c. | 77.6 | 11.6 |
| 4 | CD14 KO | 6 female mice | ACI-ACI-24 0.2 ml A | 24-0709-A | s.c. | 77.6 | 11.6 |
| 5 | TLR4 KO | 6 female mice | ACI-ACI-24 0.2 ml | 24-0709-A | s.c. | 77.6 | 11.6 |
| 5 | C57BL/6 wildtype | 6 female mice | Aβ1-15 0.2 ml | Lot 10105 33 | s.c. | 80 | N/A^{d} |
| 6 | C57BL/wildtype | female mice | PBS | N/A | s.c. | N/A | N/A |

| | | | | | | | |
|---|---|---|---|---|---|---|---|
| ^{a}: theoretical volume ^{b}: s.c.: subcutaneous ^{c}: measured quantity determined after analysis ^{d}: Not applicable | | | | | | | |

### 5.2 Results

### 5.2.1 Antibody response analysis

The anti-Aβ IgG titers of Wt, nude and CD14, CD40L or TLR4 KO mice were analyzed to verify whether the response induced by ACI-24 was dependent on T cells and/or on CD14, CD40L or TLR4.

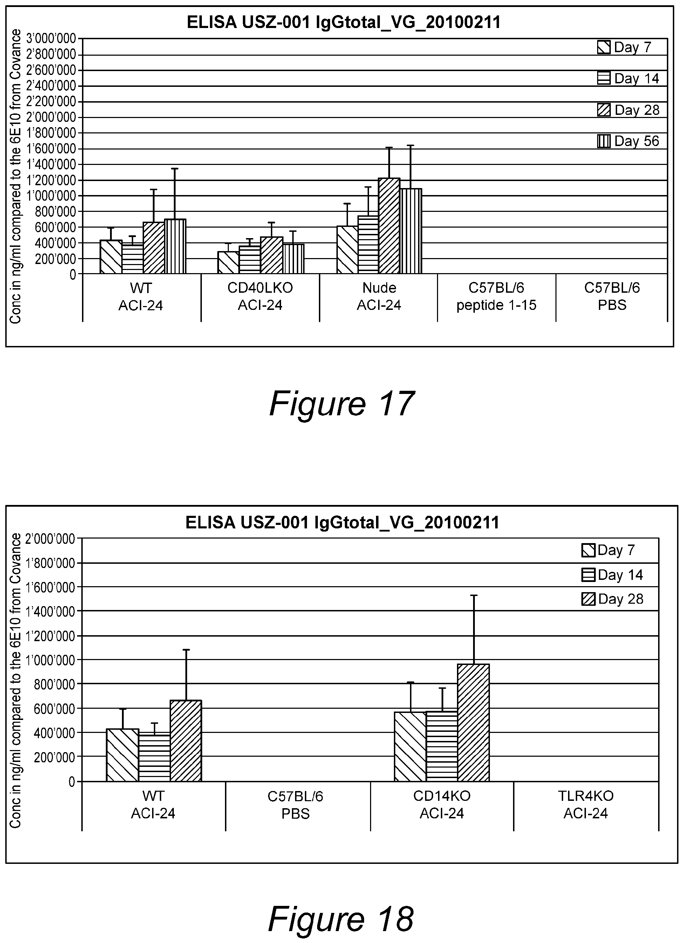

In Wt mice, the ACI-24 vaccine induced a robust anti-Aβ IgG responses in comparison to the PBS (Figure 17; 2-way ANOVA P<0.05 for d7, 28 and 56).

In CD40L KO mice ACI-24 vaccine induced a robust anti-Aβ IgG responses in comparison to the PBS (Figure 17; 2-way ANOVA P<0.05 for d7, 28 and 56) and the titers were not significantly lower than the anti-Aβ IgG titers induced in Wt mice (P<0.05 for all time points).

Following the first immunization (d7 and d14), the titers of the nude mice were similar to that of the Wt mice. At the other bleeding days, the titers of the nude mice were significantly higher than the titers of the Wt mice (2-way ANOVA P<0.001 for d28 and d56). The Aβ1-15 peptide did not induce any anti-Aβ IgG titers, as expected.

The titers in CD14 KO mice were also similar to the titers observed in Wt mice at all days analyzed (Figure 18, P> 0.05).

In contrast, no anti-Aβ IgG titers were induced after 1 or 2 immunizations with ACI-24 in TLR4 KO mice and the titers were significantly lower than the titers observed in Wt mice on both d7 ,d14 and d28 (2-way ANOVA P<0.001 on d7, d14 and d28).

Taken together, these results show that ACI-24 induces a robust antibody response that is independent on T cells, CD40L and CD14 but dependent on TLR4.

### 5.3 Conclusion

ACI-24 vaccine induced a robust anti-Aβ IgG response in Wt and nude mice demonstrating that the antibody response is independent on T-cells in the context of ACI-24 vaccination. The antibody response was also independent of CD40L further supporting the independence of T-cells. The antibody response in CD14 KO mice was also similar to Wt mice indicating that CD14 is not required as a receptor for MPLA, the adjuvant of ACI-24, to induce an antibody response. Finally, the requirement of TLR4 for ACI-24 induced antibody response was clearly demonstrated by the complete absence of antibody response in TLR4 KO mice.

### EXAMPLE 6: Anti-pTau antibody response in female nude mice

The objective of this study was to evaluate the anti-pTau antibody response induced by injection of ACI-33 (Tau5-20 [pY18]) vaccine in female nude mice. The nude mice carry the Foxn1^{nu} mutation, have a reduced T cell function due to the lack of properly functioning thymic gland. Thus, the aim of this study was to analyze whether the antibody response induced by ACI-33 is T-cell independent.

Nude mice with a C57BL/6 background and corresponding wild-type littermates at an age of 11 or 13 weeks were injected subcutaneously (s.c.). Mice were immunized 3 times with 2-week intervals and were bled 1 week after each immunization. Total anti-pTau (Tau5-20 [pY18]) peptide IgG responses were measured by ELISA. In addition, the isotype pattern of the antibody response was analyzed after 3 immunizations to evaluate the distribution of the different subclasses of IgGs as well as IgM. Antibody titers against corresponding non-pTau (Tau5-20), full-length (441aa) Tau protein and phosphorylated full-length (441aa) Tau protein were also analyzed.

To verify the absence of T-helper cells in the nude mice, the percentage of CD3⁺/CD4⁺ cells was evaluated by fluorescence-activated cell sorter (FACS).

### 6.1 Methods

### 6.1.1 Preparation of the vaccine ACI-33

The lipids Dimyristoyl phosphatidylcholine (DMPC), Dimyristoyl phosphatidylglycerol (DMPG), Cholesterol, as well as adjuvant Monophosphoryl Lipid A (MPLA) (all Avanti Polar Lipids Inc. AL, USA) and Tau-derived tetrapalmitoylated phosphopeptide Tau5-20 [pY18] (human Tau 5-20 with phospho group on Y18; 5.2 mg) were weighed into a 250 ml glass round-bottom flask (molar ratio 9:1:7:0.2:0.1, respectively). Chloroform was then added (30 ml) and after gentle agitation at RT the solution was evaporated under reduced pressure at 40 °C and then under high vacuum for 1 hour. The resulting thin-film was rehydrated by addition of PBS (60 ml) and gentle agitated at RT for 18 hours. The liposomal suspension (batch ACI-33-090818-A) was then aliquoted prior to storage at 2-8°C. The final peptide / phospholipid molar ratio was 1:100. Vaccines were shipped to JSW Life Sciences GmbH (Austria).

### 6.1.2 Immunizations

At JSW Life Sciences GmbH nude mice (B6.Cg-Foxn1nu/J) with a C57BL/6 background and corresponding wild-type littermates (6 ♀ mice/group) received s.c. injections of ACI-33 on three occasions with a 2-week interval between each administration (day 0, 14, 28) according to Table 6. Plasma samples from the facial vein/artery were collected 7 days before and 2, 4, 7, 21, 35 and 56 days after the first injections. Tau5-20 [pY18]-specific IgG and IgM antibody titers and IgG isotype patterns were determined by ELISA. Specific IgG antibodies titers for non-pTau5-20, full-length (441aa) Tau protein and phosphorylated full-length (441aa) Tau protein was also determined by ELISA. Blood samples were also collected on d-7 for FACS analysis to determine the percentage of CD3+/CD4+ cells.

### 6.1.3 Quantification of Tau peptide-specific antibodies

Specific IgG antibodies for Tau5-20 [pY18] were measured by ELISA in 5 sera bleeding samples (d2, d7, d21, d35 and d56). Tau5-20-, full-length (441aa). Tau protein- and phosphorylated full-length (441aa) Tau protein-specific IgG were determined in the sera from d35. Tau5-20 [pY18]-specific IgM and IgG isotype antibodies were determined by ELISA in the d35 sera bleeding sample. Plates were coated with 10 ug/ml of corresponding Tau peptide and 1 ug/ml of corresponding Tau protein overnight at 4°C. After washing each well with PBS-0.05% Tween 20 and blocking with 1% BSA in PBS-0.05% Tween 20, serial dilutions of sera were added to the plates and incubated at 37°C for 2 hours. After washing plates were incubated with an alkaline phosphatase (AP)-conjugated anti-mouse IgG total antibody (Jackson Laboratories, Baltimore, PA, USA) or isotype specific antibodies (horseradish Peroxidase (HRP)-conjugated anti-mouse IgM, AP-conjugated anti-mouse IgG1, biotin-conjugated anti-mouse IgG3, purchased from Pharmingen BD San Diego, CA, USA; biotin-conjugated anti-mouse IgG2a purchased from Invitrogen CA, USA and HRP-conjugated anti-mouse IgG2b from Zymed Laboratories, San Francisco, CA) for 2 hours at 37°C. After washing plates were incubated with pNPP (para-nitro-phenyl-phosphate), the phosphatase substrate for AP, or ABTS (2,2'-azino-bis(3-ethylbenzthiazoline-6-sulphonic acid)), the substrate for HRP and read at 405 nm using an ELISA plate reader. A supplemental step was done for the biotin conjugated antibodies where plates were incubated for 45 min in streptavidin-HRP (R&D Systems, Minneapolis, MN, USA) before detection using ABTS. Results are expressed as O.D. (Optical Density) at a non-saturated O.D. for IgG, IgG isotypes and IgM.

### 6.1.4 CD3+/CD4+ cell quantification

Mouse blood samples were lysed with ammonium chloride until cleared, then centrifuged at 400x g for 7 minutes and pellets were resuspended in PBS containing EDTA. Then cells were blocked with CD16/CD32 blocking reagent and stained with CD4 (PE conjugate) and CD3 (PE-Cy5) antibodies for 30 min at 4°C. Samples were washed with PBS, resuspended in fixative solution (DB Cellfix diluted 1:40 in BD FACS Flow) and acquired on a BD FACS Calibur cytometer. The percentage of gated cells, which stained positive for CD3+ and CD4+ (T-helper cells) was evaluated.

**Table 6: Mice Immunization**

| **Group** | **Numbe r of Animal s and Gende r** | **Treatm ent/ Volume ^{a}** | **Vaccine Batch** | **Proce ss** | **Route of Administration^{b}** | **Dose level Quantity of T1 ug/dose^{c}** | **Quantit y of MPLA ug/dose ^{c}** |
|---|---|---|---|---|---|---|---|
| 1 | 6 ♀ nude mice | ACI-33 0.2 ml | ACI-33-090818-A | ACI-A | s.c. | 12.6 | 15.8 |
| 2 | 6 ♀ Wt mice | ACI-33 0.2ml | ACI-33-090818-A | ACI-A | s.c. | 12.6 | 15.8 |

| | | | | | | | |
|---|---|---|---|---|---|---|---|
| *^{a}*: *theoretical volume* *^{b}: s.c.: subcutaneous* *^{c}: measured quantity determined after analysis* | | | | | | | |

### 6.2 Results

### 6.2.1 General Observations

None of the animals died prematurely and no side effects due to the treatment were reported. For all B6.Cg-Foxn1nu/J animals, the typical nude phenotype was present, while the wild-type (wt) littermates had a normal fur.

### 6.2.2 CD3+/CD4+cell quantification

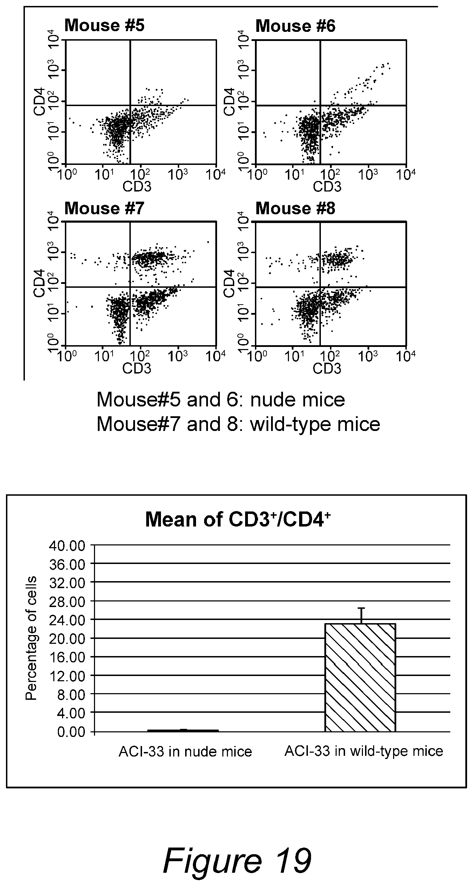

CD3+/CD4+ staining followed by FACS analysis revealed significant reduction in T-helper cell counts (CD3+/CD4+ cells) in nude mice, compared to wt animals (Figure 19).

### 6.2.3 Immune response analysis

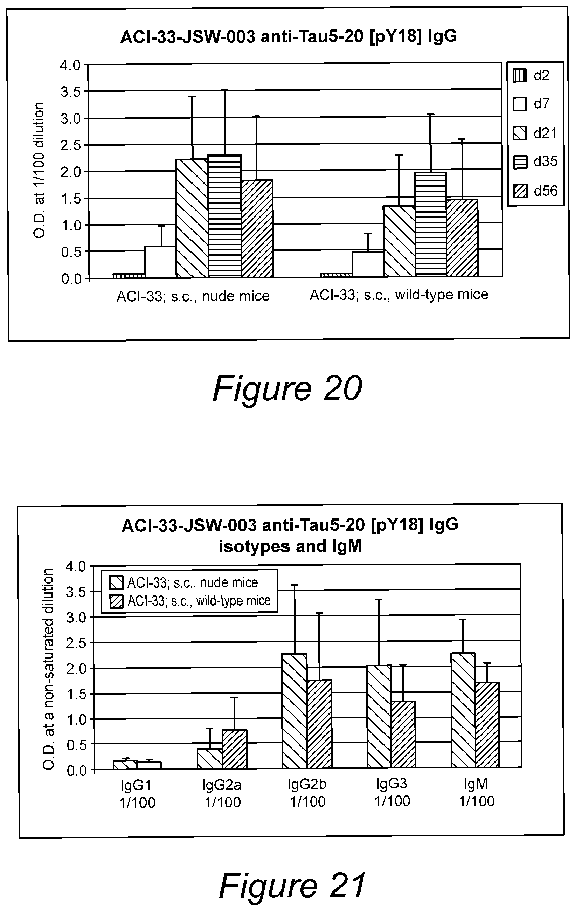

The anti-Tau5-20 [pY18] IgG titers generated by ACI-33 vaccination were analyzed to study the immunogenicity of the vaccine in wt and nude mice. The anti-Tau5-20 [pY18] IgG titers of nude were analyzed to study whether the response induced by ACI-33 was independent on T-cell function. The vaccine induced an anti-Tau5-20 [pY18] IgG response in nude mice and there was no significant difference between the antibody response induced by ACI-33 in wt or nude mice at all time points tested (Figure 20; 2-way ANOVA P<0.05 for all bleedings between nude and wt mice).

ACI-33 vaccine induced in both mouse types an anti-Tau5-20 [pY18] IgG response following s.c. injection that peaked after 2 immunizations (d27) (Figure 20).

ACI-33 vaccination induced antibody titers of the same profile for the different IgG subclass and IgM between nude and wt mice as there was no significant differences between the two mouse types following 3 s.c. immunizations of the vaccine (Figure 21, 1-way ANOVA P>0.05 IgG1 nude vs. IgG1 wt, IgG2a/2b nude vs. IgG2a/2b wt, IgG3 nude vs. IgG3 wt, IgM nude vs. IgM wt). In both mouse type there was a significant lower level of IgG1 compared to IgG2b and IgM (Figure 21, 1-way ANOVA, nude mice: P<0.01 IgG1 vs. IgG2b or IgM; Wt mice: P<0.05 IgG1 vs. IgG2b or IgM). Furthermore nude mice showed a significant lower level of IgG1 compared to IgG3 (Figure 21, 1-way ANOVA, nude mice: P<0.05 IgG1 vs. IgG3) and the level of IgG2a were also lower compared to IgG2b, IgG3 and IgM (Figure 21, 1-way ANOVA, nude mice: P<0.05 IgG2a vs. IgG2b, IgG3 or IgM).

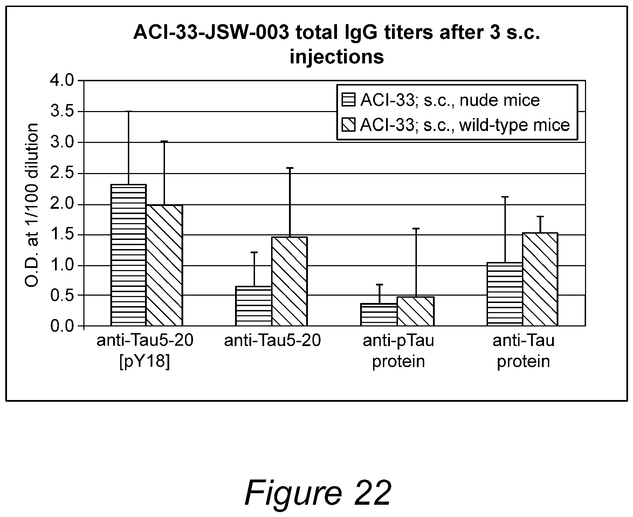

IgG titers induced after 3 s.c. injection of ACI-33 were also analyzed on different Tau peptides (anti-Tau5-20 [pY18] and anti-Tau5-20) and proteins (anti-phosphorylated full-length (441aa) Tau protein = anti-pTau protein and anti-full-length (441aa) Tau protein = anti-Tau protein (Figure 22). There was no difference in the titers on the different peptides and protein between wt and nude mice. In the nude mice group there was a significant difference in the anti-Tau5-20 [pY18] being higher then the anti-Tau5-20 titers (Figure 22, 1-way ANOVA, P<0.05 anti-Tau5-20 [pY18] titers vs. anti-Tau5-20 titers).

### 6.3 Conclusion

Despite the small percentage of CD3+ and CD4+ cells in nude mice, ACI-33 vaccine induced a robust anti-Tau5-20 [pY18] IgG response. The persistence of the antibody response and the IgG isotype distribution were similar in wt and nude mice suggesting that these parameters are independent on T cells in the context of ACI-33 vaccination. Compared to immune-competent mice, ACI-33 immunization induced an identical antibody titer and kinetic with similar IgG profile in T-cell deficient mice. Furthermore the antibody titers on the different Tau peptides and proteins were similar between immune-competent and T-cell deficient mice. These data indicated that ACI-33 induced a T-cell-independent antibody response in both nude and wt mice.

### The invention relates to, inter alia, the following embodiments:

1. An antigenic composition comprising a modified antigen for inducing a T-cell independent immune response upon treatment of a disease, condition or disorder in a patient in need of such a T-cell independent response, wherein said antigen is presented in a highly repetitive array on the surface of a liposome.
2. The antigenic composition of embodiment 1 for use in an animal or a human.
3. The antigenic composition of embodiments 1 or 2, wherein said composition is effective as an immune stimulant.
4. The antigenic composition of any one of embodiments 1 to 3, wherein said patient is a immune tolerant patient.
5. The antigenic composition of any one of embodiments 1 to 3, wherein said patient is an immunocompromised patient.
6. The antigenic composition of embodiment 5, wherein said patient has an immune system that has been impaired by age, disease such as HIV, or cancer, or by treatment such as, for example, treatment against inflammatory diseases including, but not limited to, Rheumatoid Arthritis, Psoriasis, Systemic Lupus Erythrematosis, Wegener's Granulamatosis, etc.
7. The antigenic composition of any one of embodiments 1 to 3, wherein said patient is suffering from an autoimmune disease.
8. The antigenic composition of any one of embodiments 1 to 3, wherein said patient is a T-cell activated patient.
9. The antigenic composition of any of the preceding embodiments, wherein said patient suffers from a T-cell deficiency.
10. The antigenic composition of embodiment 9, wherein said patients are depleted of CD4 T-cells.
11. The antigenic composition of embodiment 10, wherein said patients show reduced expression of CD14 and/or the CD40L on CD4 T-cells.
12. The antigenic composition of any one of embodiments 9 to 11, wherein said T-cell deficiency is caused by age, immunosuppression or disease.
13. The antigenic composition of embodiment 12, wherein said disease is HIV, cancer or other malignancies.
14. The antigenic composition according to any of the preceding embodiments, wherein the antigen does not contain T-cell epitopes.
15. The antigenic composition according to any of the preceding embodiments, wherein the antigen is a conformational antigen all or part of which comprises a beta-sheet conformation.
16. The antigenic composition according to any of the preceding embodiments, wherein the antigen is a peptide antigen, which is modified by a lipophilic or hydrophobic moiety that facilitates insertion into the lipid bilayer of a liposome carrier/adjuvant.
17. The antigenic composition according to embodiment 16, wherein the dimension of the lipophilic or hydrophobic moiety in combination with the overall net charge of the antigenic peptide and of the carrier/adjuvant to which the peptide becomes attached to, incorporated or reconstituted in is such that the antigenic peptide is exposed, stabilized and presented in a highly biologically active conformation, which allows the immune system of the target organism to freely interact with the antigenic determinants contained in the antigenic construct in its exposed, stabilized and highly biologically active conformation, which leads to a strong immune response.
18. The antigenic composition according to any one of embodiments 16 to 17 wherein the lipophilic or hydrophobic moiety is a fatty acid, a triglycerides, diglyceride, steroid, sphingolipid, glycolipid or a phospholipid.
19. The antigenic composition according to embodiment 18, wherein the lipophilic or hydrophobic moiety is a fatty acid, particularly a fatty acid with a carbon back bone of at least 10 carbon atoms.
20. The antigenic composition according to embodiment 19 wherein the hydrophobic moiety is palmitic acid.
21. The antigenic composition according to embodiment 20, wherein the peptide antigen comprises two palmitic acid moieties.
22. The antigenic composition according to embodiment 20, wherein the peptide antigen comprises four palmitic acid moieties.
23. The antigenic composition according to any of the preceding embodiments, wherein the liposome preparation contains an adjuvant, particularly lipid A, particularly detoxified lipid A, such as monophosphoryl or diphosphoryl lipid A or alum.
24. The antigenic composition according to any one of embodiments 14 to 24, wherein said antigenic peptide is a peptide derived from an amyloid protein or amyloid-like protein such as, for example prion protein, tau protein, alpha-synuclein, huntingtin.
25. The antigenic composition according to embodiment 24, wherein the antigenic construct is an Aβ antigenic construct comprising a repetitive Aβ peptide fragment derived from the N-terminus of the Aβ peptide.
26. The antigenic composition according to embodiment 25, wherein the N-terminus of the Aβ peptide comprises
   a. Aβ 1-30;
   b. Aβ 1-20;
   c. Aβ 1-16;
27. The antigenic composition according to embodiment 25, wherein the Aβ peptide fragment comprises between
   a. 4 and 20 amino acids;
   b. 5 and 16 amino acids;
   c. 6 and 12 amino acids;
   d. 4 and 8 amino acids.
28. The antigenic composition according to embodiment 25, wherein said Aβ peptide fragment is
   a. Aβ_{1-15;}
   b. Aβ₁₋₁₆;
   c. Aβ₁₋₅.
29. The antigenic composition according to any of the preceding embodiments, wherein the antigenic construct comprises an antigenic peptide, which is a phospho-peptide mimicking a major pathological phospho-epitope of protein tau.
30. The antigenic composition of embodiment 29, wherein said tau protein is a human protein.
31. The antigenic composition of embodiment 30, wherein said tau protein has an amino acid sequence identity to SEQ ID NO: 2 of at least 95%, and has substantially the same immunogenic activity as said antigenic peptide of SEQ ID NO: 2, wherein the amino acid residue corresponding to amino acid residue 18 (P-Tyr₁₈) of SEQ ID NO: 2 is phosphorylated (T1).
32. The antigenic composition of embodiment 30, wherein said tau protein has an amino acid sequence identity to SEQ ID NO: 3 of at least 95%, and has substantially the same immunogenic activity as said antigenic peptide of SEQ ID NO: 3, wherein at least one, particularly at least 2 of amino acid residues corresponding to amino acid residues 212 (P-Thr₂₁₂) and 214 (P-Ser₂₁₄) of SEQ ID NO: 3 are phosphorylated.
33. The antigenic composition of embodiment 30, wherein said tau protein has an amino acid sequence identity to SEQ ID NO: 4 of at least 95%, and has substantially the same immunogenic activity as said antigenic peptide of SEQ ID NO: 4, wherein at least one, particularly at least 2 of amino acid residues corresponding to amino acid residues 202 (P-Ser₂₀₂) and 205 (P-Thr₂₀₅) of SEQ ID NO: 4 are phosphorylated.
34. The antigenic composition of embodiment 30, wherein said tau protein has an amino acid sequence identity to SEQ ID NO: 5 of at least 95%, and has substantially the same immunogenic activity as said antigenic peptide of SEQ ID NO: 5, wherein at least one, but especially all of amino acid residues corresponding to amino acid residues 396 (P-Ser₃₉₆) and 404 (P-Ser₄₀₄) of SEQ ID NO: 5 are phosphorylated.
35. The antigenic composition of embodiment 30, wherein said tau protein has an amino acid sequence identity to SEQ ID NO: 6 of at least 95%, and has substantially the same immunogenic activity as said antigenic peptide of SEQ ID NO: 6, wherein at least one, but especially all of amino acid residues corresponding to amino acid residues 404 (P-Ser₄₀₄) and 409 (P-Ser₄₀₉) of SEQ ID NO: 6 are phosphorylated.
36. The antigenic composition of embodiment 30, wherein said tau protein has an amino acid sequence identity to SEQ ID NO: 7 of at least 95%, and has substantially the same immunogenic activity as said antigenic peptide of SEQ ID NO: 7, wherein at least one, particularly at least 2, particularly a least 3, but especially all of amino acid residues corresponding to amino acid residues 202 (P-Ser₂₀₂), 205 (P-Thr₂₀₅), 212 (P-Thr₂₁₂), and 214 (P-Ser₂₁₄) of SEQ ID NO: 7 are phosphorylated.
37. The antigenic composition of embodiment 30, wherein said tau protein has an amino acid sequence of SEQ ID NO: 2.
38. The antigenic composition of embodiment 30, wherein said tau protein has an amino acid sequence of SEQ ID NO: 3.
39. The antigenic composition of embodiment 30, wherein said tau protein has an amino acid sequence of SEQ ID NO: 4.
40. The antigenic composition of embodiment 30, wherein said tau protein has an amino acid sequence of SEQ ID NO: 5.
41. The antigenic composition of embodiment 30, wherein said tau protein has an amino acid sequence of SEQ ID NO: 6.
42. The antigenic composition of embodiment 30, wherein said tau protein has an amino acid sequence of SEQ ID NO: 7.
43. The antigenic composition of any of embodiments 24 - 28, wherein said patient is suffering from a disease or disorder which is caused by or associated with amyloid or amyloid-like proteins.
44. The antigenic composition of any of embodiments 29 - 42, wherein said patient is suffering from a disease or disorder which is caused by or associated with tau or tau-like proteins.
45. The antigenic composition according to embodiment 43, wherein the amyloid-associated disease or disorder is characterized by a loss of cognitive memory capacity in an individual, particularly animal or human.
46. The antigenic composition of embodiment 45, wherein said disease is a disease or disorder form the amyloidosis group.
47. The antigenic composition of embodiment 46, wherein said disease s a neurological disorder.
48. The antigenic composition of embodiment 47, wherein said disease is a disease or disorder selected from the group consisting of Alzheimer's Disease (AD), mild cognitive impairment (MCI), Lewy body dementia, Down's syndrome, hereditary cerebral hemorrhage with amyloidosis (Dutch type); the Guam Parkinson-Dementia complex; progressive supranuclear palsy, multiple sclerosis; Creutzfeld Jacob disease, Parkinson's disease, HIV-related dementia, ALS (amyotropic lateral sclerosis), Adult Onset Diabetes; senile cardiac amyloidosis; endocrine tumors, stroke, traumatic brain injury, macular degeneration and glaucoma.
49. The antigenic composition of embodiment 48, wherein said disease is Alzheimer's disease.
50. The antigenic composition according to embodiment 44, wherein the tau-associated disease or disorder is a neurodegenerative disorder.
51. The antigenic composition of embodiment 50, wherein said neurodegenerative disease or disorder is caused by or associated with the formation of neurofibrillary lesions.
52. The antigenic composition according to embodiment 51, wherein neurodegenerative disease or disorder is a tauopathy.
53. The antigenic composition of embodiment 52, wherein said neurodegenerative disease or disorder is one selected from the group consisting of Alzheimer's Disease, Creutzfeld-Jakob disease, Dementia pugilistica, Down's Syndrome, Gerstmann-Straussler-Scheinker disease, inclusion-body myositis, and prion protein cerebral amyloid angiopathy, traumatic brain injury, amyotrophic lateral sclerosis/parkinsonism-dementia complex of Guam, Non-Guamanian motor neuron disease with neurofibrillary tangles, argyrophilic grain dementia, corticobasal degeneration, diffuse neurofibrillary tangles with calcification, frontotemporal dementia with parkinsonism linked to chromosome 17, Hallevorden-Spatz disease, multiple system atrophy, Niemann-Pick disease, Type C, Pick's disease, progressive subcortical gliosis, progressive supranuclear palsy, Subacute sclerosing panencephalitis, Tangle only dementia, Postencephalitic Parkinsonism, Myotonic dystrophy.
54. The antigenic composition of embodiment 53, wherein said neurodegenerative disorder is Alzheimer's disease.
55. The antigenic composition of any of the preceding embodiments, wherein the individual, particularly animal or human does not show any adverse inflammatory effect.
56. A method of using an antigenic composition comprising a modified antigen according to any of the preceding embodiments for the preparation of a medicament for use in the treatment of a disease, condition or disorder in an immune tolerant patient, particularly an animal or a human, to overcome or reduce immune tolerance in said patient, wherein said antigen is presented in a highly repetitive array on the surface of a liposome.
57. A method of using an antigenic composition comprising a modified antigen according to any of the preceding embodiments for the preparation of a medicament for use in the treatment of a disease, condition or disorder in a T-cell activated patient, particularly an animal or a human, to avoid an overstimulation of the T-cell response in said patient, wherein said antigen is presented in a highly repetitive array on the surface of a liposome.
58. A method for overcoming or reducing an immune tolerance in a patient upon treatment of a disease, condition or disorder in said patient comprising administering to said patient an antigenic composition comprising a modified antigen according to any of the preceding embodiments, wherein said antigen is presented in a highly repetitive array on the surface of a liposome.
59. A method for avoidance of overstimulation of the T-cell response in a patient upon treatment of a disease, condition or disorder in said patient comprising administering to said patient an antigenic composition comprising a modified antigen according to any of the preceding embodiments, wherein said antigen is presented in a highly repetitive array on the surface of a liposome.
60. The method of embodiments 58 or 59 further comprising the step of determining the immune response in said individual by measuring the antibody concentration of IgG and/or IgM.
61. A method for inducing a T-cell independent immune response in an immune tolerant patient which suffers from a T-cell deficiency upon treatment of a disease, condition or disorder in said patient, comprising administering to said patient an antigenic composition comprising a modified antigen according to any of the preceding embodiments, wherein said antigen is presented in a highly repetitive array on the surface of a liposome.
62. A method for inducing a T-cell independent immune response in a T-cell activated patient upon treatment of a disease, condition or disorder in said patient comprising administering to said patient an antigenic composition comprising a modified antigen according to any of the preceding embodiments, wherein said antigen is presented in a highly repetitive array on the surface of a liposome.

## Claims

1. An antigenic composition comprising an adjuvant and a tau peptide antigen modified by four palmitic acid moieties inserted into the lipid bilayer of a liposome and presented in a highly repetitive array on the surface of the liposome, for use in the treatment of a tau-associated disease or disorder, in a patient having a T-cell deficiency, wherein
(i) the tau peptide is the peptide shown in SEQ ID NO:5; and
(ii) the adjuvant is MPLA.

2. The antigenic composition for use of claim 1, wherein said patient is a human.
